# FASCICULE DE BREVET EUROPEEN

(11) **EP 0 676 409 B1**
(45) Date de publication et mention de la délivrance du brevet: **19.03.2003**
(21) Numéro de dépôt: 95400763.9
(22) Date de dépôt: 06.04.1995
(51) Int. Cl.: C07H 17/08, A61K 31/70

(54) **Nouveaux dérivés de l'érythromycine, leur procédé de préparation et leur application comme médicaments**
Erythromycin-Derivate, Verfahren zur Herstellung und ihre Verwendung als Arzneimittel
Erythromycin derivatives, their process for preparation and their application as medicaments

(30) Priorité: 08.04.1994 FR 9404154
(43) Date de publication de la demande: 11.10.1995
(73) Titulaire: Aventis Pharma S.A., 92160 Antony (FR)
(72) Inventeur: Agouridas, Constantin, F-94130 Nogent sur Marne (FR); Benedetti, Yannick, F-93110 Rosny Sous Bois (FR); Chantot, Jean-François, F-94130 Nogent sur Marne (FR); Denis, Alexis, F-75011 Paris (FR); Le Martret, Odile, F-75016 Paris (FR)

(56) Documents cités:
- EP-A- 0 009 683
- EP-A- 0 487 411
- EP-A- 0 596 802
- EP-A- 0 606 024
- EP-A- 0 606 062
- EP-A- 0 614 905
- FR-A- 2 692 579
- JOURNAL OF ORGANIC CHEMISTRY., vol. 53, EASTON US, page 2340 W.R.BAKER ET AL. 'Modification of Macrolide Antibiotics. Synthesis of 11-deoxy-11-(carboxyamino)-6-O-methyleryth romycin A 11,12-(cyclic esters) via an Intramolecular Michael Reaction of O-Carbamates with an alpha,beta-Unsaturated Ketone.'
- SOMASUNDERAM A. ET AL.: 'Use of Rhodium on Carbon and 1,3-bis(diphenylphosphino)propane to Catalyze the Regioselective Hydroformylation of Alkenes with Formic Acid as the Hydrogen Source.' JOURNAL OF MOLECULAR CATALYSIS vol. 92, no. 1, 1994, pages 35 - 40, XP000862900
- HASHIZUME H. ET AL.: 'Synthesis and Biological Activity of new 3-Hydroxy-3-methylglutaryl Coenzyme A (HMG-CoA) Synthase Inhibitors : 2-Oxetanones with a Side Chain Mimicking the Folded Structure of 1233A.' CHEMICAL AND PHARMACEUTICAL BULLETIN vol. 42, no. 3, 1994, pages 512 - 520

## Description

La présente invention concerne de nouveaux dérivés de l'érythromycine, leur procédé de préparation et leur application comme médicaments.

L'invention a pour objet les composés de formule générale (I) : 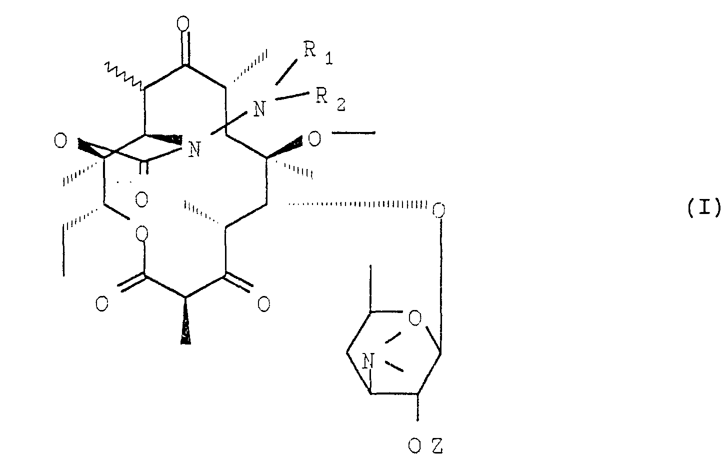 dans lesquels :
ou bien R₁ et R₂ identiques ou différents représentent un atome d'hydrogène, ou un radical hydrocarboné renfermant jusqu'à 24 atomes de carbone, saturé ou insaturé, éventuellement interrompu par un ou plusieurs hétéroatomes et portant éventuellement un ou plusieurs groupements fonctionnels,
ou bien R₁ et R₂ forment avec l'atome d'azote auquel ils sont liés un hétérocycle renfermant éventuellement un ou plusieurs hétéroatomes choisis parmi l'azote, l'oxygène et le soufre,
ou bien R₁ et R₂ forment ensemble un radical 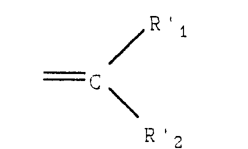 dans lesquels R'₁ et R'₂ identiques ou différents représentent un atome d'hydrogène ou un radical hydrocarboné renfermant jusqu'à 23 atomes de carbone, saturé ou insaturé, éventuellement interrompu par un ou plusieurs hétéroatomes et portant éventuellement un ou plusieurs groupements fonctionnels et Z représente un atome d'hydrogène ou le reste d'un acide carboxylique renfermant jusqu'à 18 atomes de carbone, le trait ondulé en position 10 indiquant que le méthyl peut être de configuration R ou S, ou un mélange de configuration R et S, ainsi que les sels d'addition avec les acides des composés de formule (I).

Parmi les sels d'addition avec les acides, on peut citer les sels formés avec les acides acétique, propionique, trifluoroacétique, maléique, tartrique, méthanesulfonique, benzènesulfonique, p-toluènesulfonique et spécialement les acides stéariques, éthylsuccinique ou laurylsulfonique.

Le radical hydrocarboné que peut représenter R₁ ou R₂ et R'₁ ou R'₂ peut être interrompu par un ou plusieurs hétéroatomes choisis parmi l'azote, l'oxygène et le soufre et peut porter un ou plusieurs groupements choisis dans le groupe constitué par les radicaux hydroxyle, les atomes d'halogène, les radicaux NO₂, les radicaux C≡N, les radicaux alkyle, alkényle ou alkynyle, O-alkyle, O-alkényle ou O-alkynyle, S-alkyle, S-alkényle ou S-alkynyle et N-alkyle, N-alkényle ou N-alkynyle, renfermant jusqu'à 12 atomes de carbone éventuellement substitués par un ou plusieurs atomes d'halogène, le radical 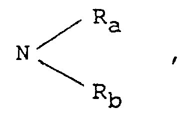 Rₐ et R_{b} identiques ou différents, représentant un atome d'hydrogène ou un radical alkyle renfermant jusqu'à 12 atomes de carbone, le radical 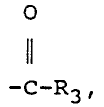 R₃ représentant un radical alkyle renfermant jusqu'à 12 atomes de carbone, ou un radical aryle ou hétéroaryle à 5 ou 6 chaînons comportant un ou plusieurs hétéroatomes, éventuellement substitués par un ou plusieurs des substituants mentionnés ci-dessus.

Le radical hydrocarboné que peut représenter R₁ ou R₂ et R'₁ ou R'₂ peut être un radical alkyle, alkényle, alkynyle, aralkyle, aralkényle ou aralkynyle. Dans la définition des substituants, le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, propargyle, cyclobutyle, cyclopentyle ou cyclohexyle.

Le radical aryle peut être un radical phényle ou naphtyle.

Le radical aryle peut être également un radical hétérocyclique substitué ou non comme le radical thiényle, furyle, pyrolyle, thiazolyle, oxazolyle, imidazolyle, thiadiazolyle, pyrazolyle ou isopyrazolyle, un radical pyridyle, pyrimidyle, pyridazinyle ou pyrazinyle, ou encore un radical indolyle benzofurannyle, benzothiazyle ou quinoléinyle. Ces radicaux aryles peuvent comporter un ou plusieurs des groupements mentionnés ci-dessus.

Lorsque R₁ et R₂ forment avec l'atome d'azote auquel ils sont liés un radical hétérocyclique, il s'agit de préférence d'un radical pyrrolyle, pyrrolidinyle, pyridyle, pyrazinyle, pyrimidyle, pipéridinyle, pipérazinyle, quinuclidinyle, oxazolyle, isoxazolyle, morpholinyle, indolyle, imidazolyle, benzimidazolyle, triazolyle, thiazolyle, azétidinyle, aziridinyle.

L'invention a plus particulièrement pour objet, les composés de formule (I) dans lesquels Z représente un atome d'hydrogène, les composés de formule (I) dans lesquels R₁ représente un atome d'hydrogène, les composés de formule (I) dans lesquels R₁ et R₂ représentent chacun un atome d'hydrogène.

Parmi les composés préférés de l'invention, on peut citer les composés de formule (I) dans lesquels R'₁ représente un atome d'hydrogène, on peut citer les composés dans lesquels R₁ et R₂ forment ensemble un radical :

=CH(CH₂)ₙAr₁

dans lequel Ar₁ représente un radical aryle ou hétéroaryle éventuellement substitué et n représente un nombre entier pouvant varier de 0 à 8.

Ar₁ a comme valeur préférée, l'une ou l'autre des valeurs préférées indiquées ci-dessus pour les radicaux aryle et hétéroaryle. Le ou les substituants éventuels de Ar₁ sont ceux indiqués ci-dessus comme groupements fonctionnels.

Parmi les composés préférés de l'invention, on peut citer les composés de formule (I) dans lesquels R₁ et R₂ forment ensemble un radical : 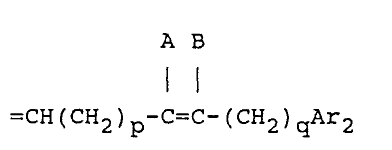 dans lequel p et q identiques ou différents, représentent un nombre entier variant de 0 à 6, A et B identiques ou différents représentent un atome d'hydrogène ou d'halogène ou un radical alkyle renfermant jusqu'à 8 atomes de carbone, la géométrie de la double liaison étant E ou Z ou un mélange E + Z, ou bien A et B forment une troisième liaison avec les atomes de carbone auxquels ils sont liés et Ar₂ représente un radical aryle ou hétéroaryle, mono ou polycyclique, éventuellement substitué.

Parmi ces composés on peut citer tout spécialement les composés dans lesquels p et q représentent le nombre 0, ainsi que ceux dans lesquels A et B représentent un atome d'hydrogène.

Parmi les composés préférés de l'invention, on peut citer les composés de formule (I) dans lesquels R₂ représentent un radical :

(CH₂)ᵣAr₃

dans lesquels r représente un nombre entier variant de 0 à 6 et Ar₃ représente un radical aryle ou hétéroaryle éventuellement substitué, et plus particulièrement ceux dans lesquels Ar₃ représente un radical 4-quinoléinyle éventuellement mono ou polysubstitué sur l'un et/ou l'autre des 2 cycles de la quinoléine, par exemple ceux dans lesquels Ar₃ représente un radical 4-quinoléinyle non substitué, 4-quinoléinyl substitué par un radical méthoxy ou un radical thiazolyle substitué par un radical pyridinyle.

L'invention a tout particulièrement pour objet les composés de formule (I) dans lesquels r représente un nombre entier variant de 1 à 4.

Parmi les composés préférés de l'invention, on peut citer les composés dont la préparation est donnée ci-après dans la partie expérimentale et plus particulièrement les composés dont les noms suivent :
- 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl (2-(3-(4-quinoléinyl) propyl) hydrazono)) érythromycine,
- 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl (2-(3-(7-méthoxy-4-quinoléinyl) propyl) hydrazono)) érythromycine,
- 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl (2-(3-(2-(3-pyridinyl-4-thiazolyl) propyl) hydrazono)) érythromycine.

Les produits de formule générale (I) possèdent une très bonne activité antibiotique sur les bactéries gram ⊕ telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppuration pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma, ou à des germes du genre Mycobactérium.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits des exemples 5 ou 11, 12 et 13 et leurs sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 5.

L'invention a également pour objet un procédé de préparation des composés de formule (I), caractérisé en ce que l'on soumet un composé de formule (II) : 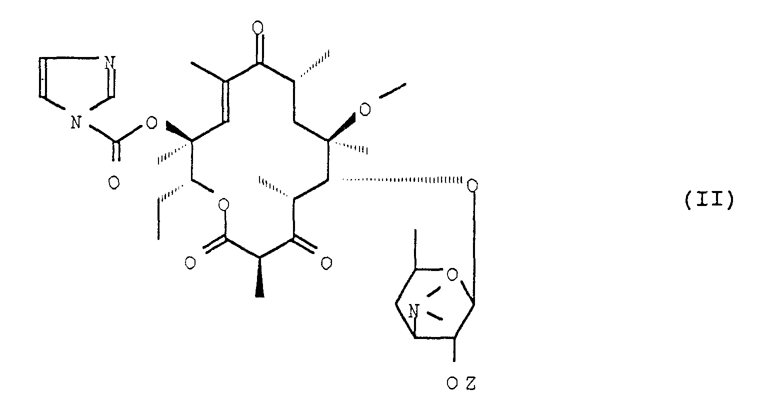 dans laquelle Z conserve sa signification précédente, ou bien à l'action de l'hydrazine NH₂NH₂ pour obtenir le composé de formule (I_{A}) : 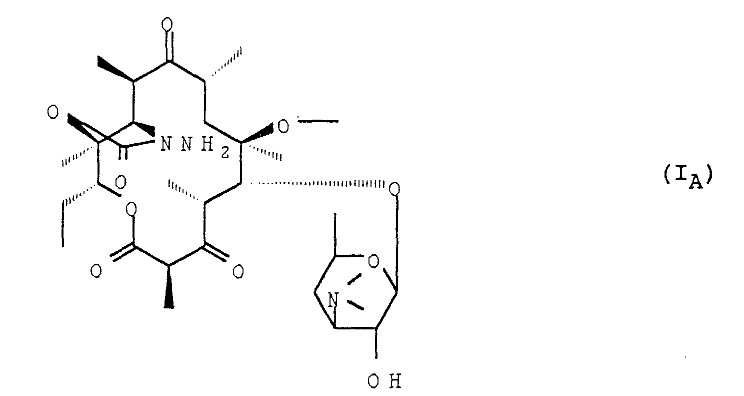 que l'on soumet si désiré à l'action d'un aldéhyde R'₂CHO ou d'une cétone 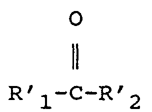 dans lesquels R'₁ et R'₂ ont la signification indiquée ci-dessus, pour obtenir le composé de formule (I_{B}) correspondant : 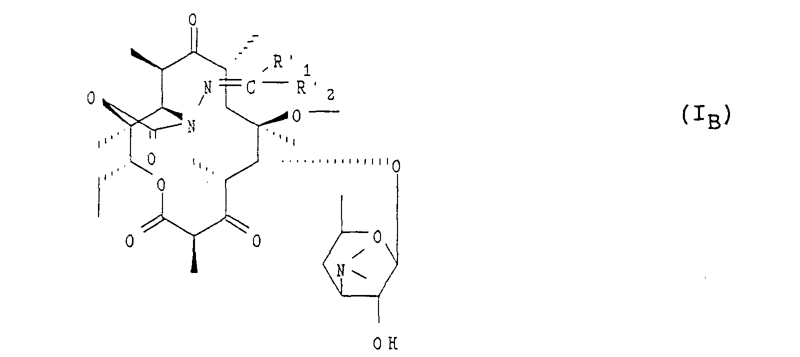 dans laquelle R'₁ et R'₂ conservent la même signification que précédemment, que l'on soumet si désiré à l'action d'un agent de réduction pour obtenir le composé de formule (I_{C}) correspondant : 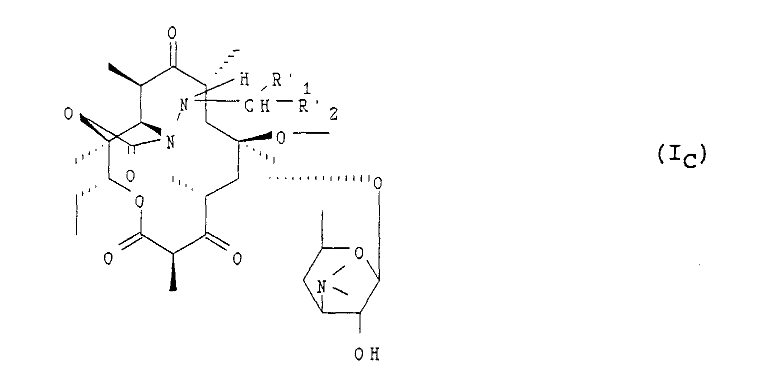 dans laquelle R'₁ et R'₂ conserve sa signification précédente, c'est-à-dire un composé de formule (I) dans lequel R₁ est l'hydrogène et R₂ représente un radical CHR'₁-R'₂, puis si désiré, soumet le composé de formule (I_{C}) à l'action d'un agent susceptible de remplacer l'atome d'hydrogène du groupement NH par un groupement R₁ tel que défini précédemment à l'exception de la valeur d'hydrogène, puis si désiré, soumet le composé obtenu à l'action d'un acide pour en former le sel et/ou à l'action d'un agent d'estérification du groupement OH en 2'.

Les composés de formule (II) utilisés comme produits de départ du procédé de l'invention sont décrits et revendiqués dans la demande de brevet européen EP 0596802.

Dans un mode de réalisation préféré de l'invention :
- on opère en présence d'un excès d'hydrazine, à une température supérieure à la température ambiante par exemple une température comprise entre 40°C et 80°C, dans un solvant tel que l'acétonitrile, le dioxane, le diméthylformamide, le tétrahydrofuranne, le diméthoxy éthane ou le diméthylsulfoxyde, (en présence ou en l'absence d'une base),
- la réaction avec l'aldéhyde ou la cétone a lieu dans les mêmes conditions de température et de solvant,
- l'agent de réduction est NaBH₃CN ou l'hydrogène en présence d'un catalyseur tel que le palladium, le platine et indifféremment en présence ou en l'absence d'un acide tel que l'acide chlorhydrique ou l'acide acétique,
- l'estérification en 2' est réalisée selon les procédés classiques,
- la salification est réalisée au moyen d'acides selon les procédés classiques.

L'invention a également pour objet une variante du procédé précédent, caractérisé en ce que l'on soumet le composé de formule (II) : 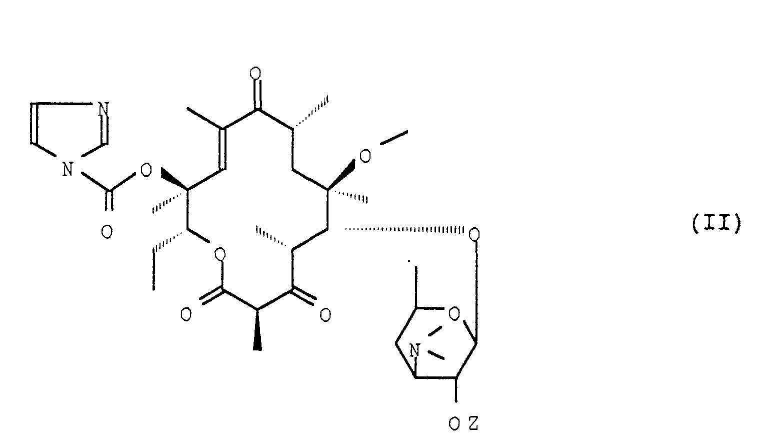 dans lequel Z conserve sa signification précédente, à l'action d'un composé de formule NH₂NHR₂ pour obtenir le composé de formule (I'_{A}) : 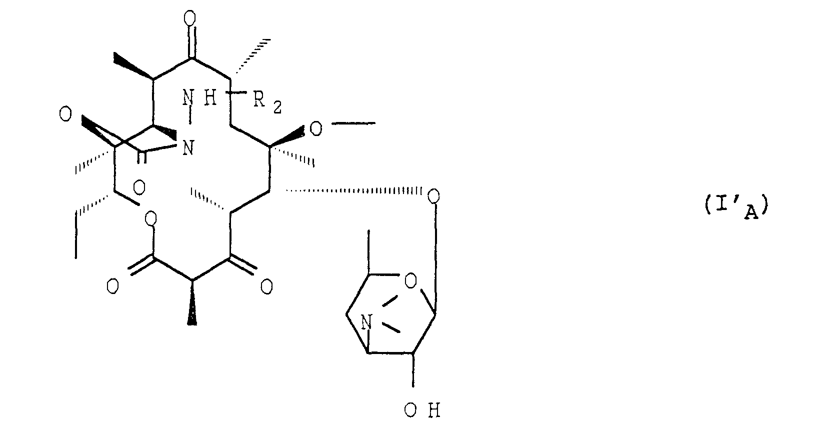 que l'on soumet si désiré, à l'action d'un agent susceptible de remplacer l'atome d'hydrogène du groupement NH par un radical R₁ tel que défini ci-dessus à l'exception de la valeur hydrogène pour obtenir le composé de formule (I'_{B}) correspondant : 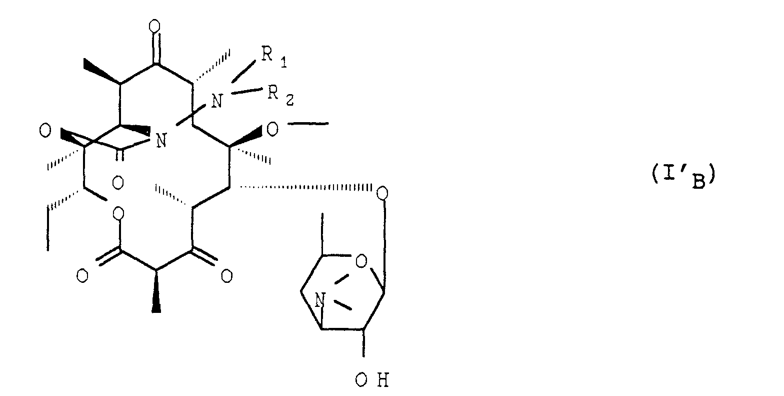 que l'on soumet si désiré, à l'action d'un agent d'estérification du groupement OH en 2' ou à l'action d'un acide pour en former le sel, les conditions préférentielles de température et de pression sont celles décrites ci-dessus.

Les exemples suivants illustrent l'invention.

### EXEMPLE 1 : 11,12-didéoxy 3-de-((2,6-didéoxy 3-C-méthyl 3-O-méthyl alpha L-ribohexopyranosyl) oxy) 6-O-méthyl 3-oxo 12,11-(oxycarbonyl (hydrozono) érythromycine isomère 10(R) et isomère 10(S) correspondant

On met en suspension dans 5 ml de cyanure de méthyle et 0,5 ml d'eau 353 mg de 2'-acétate de 11-déoxy 10,11-didéhydro 3-de(2,6-didéoxy 3-C-méthyl 3-O-méthyl alpha-L-ribohexopyranosyl) oxy) 12-O-((lH-imidazol-1-yl) carbonyl) 6-O-méthyl 3-oxo érythromycine préparé comme indiqué dans la demande de brevet européen EP 0596802 et 0,097 ml d'hydrate d'hydrazine. On chauffe le mélange réactionnel à 60°C pendant 3 heures. On verse le mélange réactionnel dans l'eau, extrait à l'acétate d'éthyle, lave et sèche. On chromatographie le mélange réactionnel sur silice en éluant avec le mélange éther isopropylique, triéthylamine, méthanol (90/10/10). On obtient 101 mg de produit recherché (produit A). Rf = 0,45 et 106 mg de produit 10(S) correspondant (produit B).

| Produit A : | | | |
|---|---|---|---|
| Analyse | % calculé | % trouvé | |
| | | Pdt (A) | Pdt (B) |
| C | 59,31 | 59,3 | 59,3 |
| H | 8,51 | 8,4 | 8,4 |
| N | 6,69 | 6,7 | 6,8 |

| Produit A : | RMN CDCl₃ ppm | Produit B : | RMN CDCl₃ ppm |
|---|---|---|---|
| 3,09 | H₁₀ (m) | 3,53 | H₁₀ (m) |
| 3,59 | H₁₁ (s) | 3,46 (d,J=3Hz) | H₁₁ (s) |
| 1,35 | 12 Me (s) | 1,32 | 12 Me (s) |
| 5,03 | H₁₃ (dd) | 4,95 | H₁₃ (dd) |
| 0,86 | 15 Me (t) | 0,87 | 15 Me (t) |
| 3,85 | H₂ (q) | 3,88 | H₂ (q) |
| 2,30 | N-Me (s) | 2,31 | N-Me (s) |
| 2,67-6 | OMe (s) | 2,83 | 6-OMe (s) |
| 4,44 | NH (s) | 3,84 | NH (s) |
| 2,67 | H₈ (m) | 2,78 | H₈ (m) |

### EXEMPLE 2 : 11,12-didéoxy 3-de(2,6-didéoxy 3-C-méthyl 3-O-méthyl alpha-L-ribohexopyranosyl) oxy) 6-O-méthyl 3-oxo 12,11(oxycarbonyl (2-(3-phénylpropylidène) hydrazono)) érythromycine

On met en solution dans 2 ml de THF sur tamis moléculaire (4Å), 285 mg de produit A obtenu à l'exemple 1 et 156 mg de 3-phénylpropionaldéhyde. On ajoute 100 m de tamis moléculaire (4Å) et chauffe à 60°C pendant 24 h. On filtre, concentre et purifie par chromatographie sur silice, en éluant avec le mélange acétate d'éthyle, triéthylamine (96-4). On recueille les fractions de rf = 0,41 et obtient 330 mg de produit recherché rf = 0,3.

| Analyse | % calculé | % trouvé |
|---|---|---|
| C | 64,58 | 64,3 |
| H | 8,26 | 8,3 |
| N | 5,65 | 5,5 |

| RMN CDCl₃ ppm | |
|---|---|
| 3,04 | H₁₀ (q) |
| 4,46 | H₁₁ (d,j = 3 Hz) |
| 5,05 | H₁₃ (dd) |
| 3,85 | H₂ (q) |
| 2,38 | NMe (s) |
| 2,79 | 6 OMe (s) |
| 7,96 | N=CH (t) |
| 2,86 | CH₂-Φ |
| 7,2,7,35 | H aromatiques |
| 2,61 | NH=CH-CH₂ (m). |

### EXEMPLE 3 : 11,12-didéoxy 3-de((2,6-didéoxy 3-C-méthyl 3-O-méthyl alpha-L-ribohexopyranosyl) oxy) 6-O-méthyl 3-oxo 12,11(oxycarbonyl (2-(3-phénylpropyl hydrazono)) érythromycine

On ajoute 23 mg de cyanoborohydrure de sodium (NaBH₃CN) dans une solution renfermant 1,5 ml de méthanol 88 mg du produit de l'exemple 2 et 50 µl d'acide acétique. On concentre, reprend à l'acétate d'éthyle, ajoute de l'eau et amène à pH 8 à l'aide d'une solution de soude 2N. On décante, lave avec une solution saturée de chlorure de sodium et sèche. On chromatographie le produit obtenu sur silice (éluant : éther isopopylique-méthanol-triéthylamine 90-10-10). On recueille les fractions de rf=0,33. On reprend le mélange obtenu dans un mélange éther-pentane et filtre. Après évaporation, on obtient 70 mg du produit attendu.

| Analyse | % calculé | % trouvé |
|---|---|---|
| C | 64,4 | 64,2 |
| H | 8,51 | 8,3 |
| N | 5,63 | 5,6 |

| RMN CDCl₃ ppm | |
|---|---|
| 3,74 | H₁₀ (s) |
| 5,03 | H₁₃ (dd) |
| 3,86 | H₂ (q) |
| 2,27 | N(CH₃)₂ (s) |
| 2,64 | 6 OMe (s) |
| 2,72 | CH₂-Φ |
| 7,13-7,28 | H aromatiques |
| 5,35 | H de NH (t). |

### EXEMPLE 4 : 11,12-didéoxy 3-de((2,6-didéoxy 3-C-méthyl 3-O-méthyl alpha-L-ribohexopyranosyl) oxy) 6-O-méthyl 3-oxo 12,11(oxycarbonyl (2-(3-(4-quinoléinyl) 2(E)-propénylidène) hydrazono)) érythromycine

On agite à la température ambiante pendant 5 heures 125 mg de produit A préparé à l'exemple 1, 73 mg de 4-quinoléinyl propénal dont la préparation est donnée ci-après et 40 µl d'acide acétique. On élimine le méthanol sous pression réduite et reprend avec un mélange chlorure de méthylène-eau. On amène à pH 9 à l'aide d'une solution concentrée d'ammoniaque. On décante, sèche sur sulfate de magnésium, filtre et évapore à sec. On obtient 211 mg d'un produit que l'on chromatographie sur silice en éluant avec un mélange chlorure de méthylène-méthanol (92-8). On empâte dans un mélange acétate d'éthyle-pentane (1-1), le produit de rf = 0,4. On essore, rince avec le minimum du mélange acétate d'éthyle-pentane, sèche en étuve sous pression réduite le produit obtenu. On obtient ainsi 109 mg du produit recherché.

### PREPARATION DE L'EXEMPLE 4 : 4-quinoléine propénal

On met en solution dans 80 ml de chlorure de méthylène, 3,9 g de 4-quinoléine carboxaldéhyde. On refroidit à 10°C ± 5°C et ajoute en 1 h 30 et en maintenant la température à 10°C, 8,3 g de 3-(triphénylphosphine) propénal (C₆H₅)₃P=C-CHO. On laisse la température revenir à 20°C et poursuit l'agitation pendant 24 heures. On refroidit à nouveau vers 10°C et ajoute 0,4 g de (C₆H₅)₃P=C-CHO. On agite encore pendant 3 heures à la température ambiante. On évapore le chlorure de méthylène et obtient un produit que l'on chromatographie sur silice en éluant avec le mélange acétate d'éthyle-cyclohexane (4-6). On isole 2,12 g de produit recherché. F ∼ 90°C.

### EXEMPLE 5 : 11,12-didéoxy 3-de((2,6-didéoxy 3-C-méthyl 3-O-méthyl alpha-L-ribohexopyranosyl) oxy) 6-O-méthyl 3-oxo 12,11(oxycarbonyl (2-(3-(4-quinoléinyl) 2-propyl) hydrazono)) érythromycine

On met en solution dans 10 ml d'acétate d'éthyle, 0,38 g de produit préparé à l'exemple 4 et 38 mg d'oxyde de platine. On hydrogène sous vive agitation pendant 24 heures. On filtre, rince à l'acétate d'éthyle et évapore sous pression réduite. On obtient 0,375 g de produit que l'on reprend dans 5 ml de méthanol, 175 µl d'acide acétique et 90 mg de borohydrure de sodium. On agite 3 heures à la température ambiante. On chasse le méthanol et reprend à l'aide du mélange chlorure de méthylène-eau. On amène à pH 8-9 avec une solution d'ammoniaque à 28 %. On décante, lave à l'eau, sèche, filtre et évapore à sec. On obtient 0,37 g de produit que l'on chromatographie sur silice, en éluant avec un mélange acétate d'éthyle-triéthylamine 96-4. On obtient 127 mg d'un produit (rf = 0,25) que l'on essore, lave et sèche. On obtient 90 mg du produit recherché F = 189°C.
RMN CDCl₃ ppm, 300 MHz
1,34 (s)-1,48 (s) : 6 et 12 CH₃ ; 2,30 (s) : N(CH₃)₂ ; 2,65 (s) : 6-OCH₃ ; 3,06 (dq) : H₄ ; 3,19 (q) : H₁₀ ; 3,74 (s) : H₁₁ ; 5,50 (t. mobile) : NH-CH₂ ; 7,30 (d) : H₃ quinoléine ; 7,53-7,68 (dt) : H₆-H₇ quinoléine ; 8,10 (m) : H₅-H₈ quinoléine ; 8,79 (d) : H₂ quinoléine.

### EXEMPLE 6 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (2-(3-(lH-benzimidazol-1-yl) propyl) hydrazono)) érythromycine

On agite 18 heures à température ambiante une solution de 300 mg du produit obtenu à l'exemple 1, 168 mg de 3-imidazolyl-propanal (dont la préparation est donnée ci-après) et 90 ml d'acide acétique dans 9 ml de méthanol, on ajoute après ce temps, 40 mg de cyanoborohydrure de sodium, on agite encore 5 h puis ajoute 120 mg de cyanoborohydrure de sodium et 200 µl d'acide acétique. On poursuit l'agitation pendant 48 h, ajoute un mélange de chlorure de méthylène et d'eau, ajuste le pH à 8-9 avec de l'ammoniaque à 32 %, sépare la phase organique, sèche et évapore à sec. On obtient 0,6 g de résidu que l'on chromatographie sur silice (éluant acétate d'éthyle-méthanol-TEA : 92-6-2) le produit obtenu est empâté dans un mélange éther-pentane 1-5. On recueille 143 mg du produit recherché brut que l'on dissout dans 1 ml d'acétate d'éthyle, filtre et cristallise par addition de 3 ml de pentane, on obtient après séchage 85 mg de produit recherché (F = 197°C).

| Analyse pour C₄₁H₆₃N₅O₁₀ 785,98 | | | |
|---|---|---|---|
| | C | H | N |
| % calculés | 62,65 | 8,08 | 8,91 |
| % trouvés | 62,5 | 8,1 | 8,8 |

RMN CDCl₃ ppm
3,18 : H₁₀ ; 3,69 (s) : H₁₁ ; 0,84 (t) : 15 CH₃ ; 3,86 (q) : H₂ ; 2,45 : N-(CH₃)₂ ; 2,60 (s) : 6 OCH₃ ; 5,56 (t) : NH ; 2,65-2,81 : NH-CH₂- ; 4,50 : -CH₂-N 〈 ; 7,26 à 8,02 : 5H benzimidazole.

### PREPARATION DE L'EXEMPLE 6 : 3-imidazolyl propanal

### Stade A : 2-[2-(3-imidazolyl) éthyl] 1,3-dioxolane

A une solution de 1,2 g de benzimidazole dans 15 ml de diméthylformamide, on ajoute 0,49 g d'hydrure de sodium à 50 % en dispersion dans l'huile. La température s'élève à 35°C, dix minutes près la fin du dégagement gazeux, on ajoute en laissant la température s'élever à 35°C, 1,2 ml de 2-(2-bromoéthyl) 1,3-dioxolane. On agite deux heures, on ajoute de l'eau saturée de chlorure de sodium, extrait avec de l'éther, sèche, filtre et évapore sous pression réduite, on obtient 2 g de résidu que l'on chromatographie sur silice en éluant avec chlorure de méthylène-méthanol (95-5). On recueille ainsi 1,6 g de produit recherché.
RMN CDCl₃ :
2,25 et 4,35 : les CH₂ de l'éthyle ; 3,85 à 4,00 : les CH₂ du dioxolane ; 4,87 : CH dioxolane ; 7,29-7,45-7,81 : 4H benzimidazole ; 7,92 : H en 2 de l'imidazole.

### STADE B : 3-imidazolyl propanal

On agite 5 heures au reflux une solution de 1,6 g du produit obtenu au stade A, 1,45 g d'acide paratoluène sulfonique dans 60 ml de méthanol. On ajuste à pH 8 avec du carbonate de potassium, élimine le méthanol sous pression réduite, extrait avec du chlorure de méthylène, lave à l'eau, sèche et évapore à sec sous pression réduite, on obtient 1,45 g de diméthoxy cétal intermédiaire que l'on agite à 40°C pendant 18 h, en présence de 70 ml d'acétone et de 34 ml d'acide chlorhydrique 2N, évapore l'acétone sous pression réduite et amène le pH à 8-9 par addition d'ammoniaque à 32 %, on extrait avec du chlorure de méthylène, lave à l'eau, sèche et évapore à sec sous pression réduite, on recueille 1,13 g de produit que l'on chromatographie sur silice, éluant chlorure de méthylène-méthanol 95-5. On obtient 0,796 g du produit recherché.
RMN CDCl₃ 250 MHz
3,07 (t)-4,52 (t) : les CH₂ de l'éthyle ; 7,25 à 7,50 : les aromatiques ; 9,79 (s) : CH de l'aldéhyde.

### EXEMPLE 7 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (2-(3-(2-phényl 5-thiazolyl) propyl) hydrazono)) érythromycine

On agite 4 heures à température ambiante, 200 mg du produit obtenu à l'exemple 1, 139 mg de 3-(2-phényl 5-thiazolyl) propanal (dont la préparation est donnée ci-après), 180 ml d'acide acétique et 7 ml de méthanol puis ajoute 60 mg de cyanoborohydrure de sodium. On agite 18 heures à température ambiante, évapore à sec sous pression réduite, on reprend le résidu avec un mélange eau-acétate d'éthyle et ajuste à pH 9 avec une solution aqueuse d'ammoniaque. On extrait avec de l'acétate d'éthyle, lave à l'eau, sèche, évapore à sec sous pression réduite. On recueille 354 mg de produit que l'on chromatographie sur silice, éluant acétate d'éthyle puis acétate d'éthyle-triéthylamine (96/4). On recueille 170 mg de produit que l'on cristallise dans le mélange acétate d'éthyle-pentane 1/5. On obtient ainsi 80 mg du produit recherché.

| Analyse pour C₄₃H₆₄N₄O₁₀S 829,07 | | | | |
|---|---|---|---|---|
| | C | H | N | S |
| % calculés | 62,3 | 7,78 | 6,76 | 3,87 |
| % trouvés | 62,0 | 7,8 | 6,8 | 4,0 |

RMN CDCl₃ 300 MHz
3,17 (m) : H₁₀ ; 1,07 (d) : 14 CH₃ ; 1,48 : 15 CH₃ ; 3,87 (q) : H₂ ; 2,26 (s) : N-(CH₃)₂ ; 3,53 (m) : 2'OH ; 2,67 (s) : 6-OCH₃ ; 5,43 (t) : NH ; 2,86 (m)-1,95 (m)-3,03 (m) : les CH₂ propyle ; 7,55 (s) : H du thiazole ; 7,39 (m) 3H et 7,89 (m) 2H : les aromatiques ; 1,19 (d) : H₈.

### PREPARATION DE L'EXEMPLE 7 : 2-phényl 5-thiazole propanal

### Stade A : 2-phényl 5-carbéthoxy thiazole

A une suspension de 78 g de thiobenzamide dans 200 ml de benzène, on ajoute une solution de formyl béta chloracétate d'éthyle dans 240 ml de benzène. On chauffe au reflux pendant 3 heures 30 en éliminant l'eau formée. On refroidit et ajoute lentement 320 ml d'une solution de carbonate de potassium à 20 % et 220 ml d'eau, extrait avec de l'éther, lave, sèche et distille sous pression réduite on obtient 75,5 g de produit recherché.

### Stade B : acide 2-phényl 5-thiazole carboxylique

A une solution de 75,5 g du produit obtenu au stade A, dans 130 ml d'éthanol, on ajoute 28,56 g de potasse en pastilles en solution dans 410 ml d'éthanol, on chauffe 15 minutes au reflux, refroidit et essore le sel de potassium, le lave à l'éther et sèche sous pression réduite. On obtient 53,5 g de sel de potassium intermédiaire que l'on dissout dans 1,2 litre d'eau et acidifie à pH 1 avec une solution d'acide chlorhydrique concentré après filtration, on recueille 29 g de produit recherché (F = 192°C). 24,5 g de produit sont recristallisés dans 750 ml de toluène. On obtient 20 g de produit recherché. F = 195°C.

| Analyse pour C₁₀H₇NO₂S 205,2 | | | | |
|---|---|---|---|---|
| | C | H | N | S |
| % calculés | 58,52 | 3,43 | 6,82 | 15,6 |
| % trouvés | 58,5 | 3,7 | 6,8 | 15,2 |

### Stade C : 2-phényl 5-thiazole carboxylate de méthyle

A une solution de 4,77 g de l'acide obtenu au Stade B dans 160 ml de méthanol, on ajoute 2,5 ml de chlorure d'acétyle et chauffe au reflux pendant 18 h. On amène à sec sous pression réduite, reprend avec de l'acétate d'éthyle, filtre, concentre à volume réduit et essore les cristaux obtenus. On lave les liqueurs mères avec de la soude, extrait avec de l'acétate d'éthyle, lave à l'eau et évapore à sec, on réunit les 2 fractions cristallisées pour obtenir 4,54 g de produit recherché (F = 108°C).

### Stade D : 2-phényl 5-formyl thiazol

### Réduction :

A une suspension de 1,45 g d'hydrure de lithium-aluminium dans 65 ml de tétrahydrofuranne refroidie à 10°C, on ajoute en 20 minutes et en maintenant la température à 10°C, une solution de 4,5 g du produit obtenu au stade C dans 35 ml de tétrahydrofuranne, on agite 45 minutes à 10°C, puis 2 h à température ambiante. On ajoute du tétrahydrofuranne à 10 puis 50 % d'eau en maintenant la température inférieure à 20°C, on ajoute 15 ml d'une solution de tartrate de potassium et sodium, on filtre, rince et amène à sec sous pression réduite ; le résidu obtenu est empâté dans l'hexane, essoré et séché à 40°C sous pression réduite, on recueille 3,6 g de produit. F = 82°C.

### Oxydation :

On agite pendant 2 h 30 à température ambiante, 3,57 g du produit obtenu ci-dessus avec 143 ml de toluène et 17,9 g de bi-oxyde de manganèse. On filtre et amène à sec sous pression réduite. On reprend le résidu avec de l'hexane, essore et sèche à 40°C sous pression réduite, on recueille 3,09 g de produit recherché. F = 94°C.

### Stade E : 3-(2-phényl 5-thiazolyl) propénal

On ajoute en 10 minutes 5 g de (formylméthylène) triphénylphosphorane à une solution de 2,098 g du produit obtenu au stade D, et agite pendant 27 h à température ambiante. On évapore à sec sous pression réduite et recueille 6,60 g de produit que l'on chromatographie sur silice en éluant avec acétate d'éthyle-cyclohexane (2-8). On obtient 1,22 g de produit que l'on empâte dans le pentane pour obtenir 1,047 g du produit recherché (F = 104°C).
RMN CDCl₃ (250 MHz)
8,04 (s) : H triazole ; 6,49 (ddJ = 7,5) et 7,69 (dJ = 15,5) les H propène ; 9,67 (J = 7,5) CHO ; 7,50 (m) 3H et 7,97 (m) 2 H : les aromatiques.

### Stade F : 3-(2-phényl 5-thiazolyl) propénol

A une suspension de 475 mg de borohydrure de sodium dans 50 ml d'éthanol, on additionne par portions 900 mg de l'aldéhyde obtenu au stade E ci-dessus, on agite, ensuite 20 min à température ambiante puis détruit l'excès de borohydrure de sodium en ajoutant de l'acétone. On évapore à sec sous pression réduite et reprend avec de l'acétate d'éthyle, lave avec de l'eau salée, sèche et amène à sec sous pression réduite, on obtient 960 mg de produit utilisé tel quel pour l'étape suivante.

### Stade G : 2-phényl 5-thiazolyl propanol

On hydrogène pendant 12 h sous = 1.01325 Pa (1 atmosphère) puis 9 h sous 1.41855 Pa (1.4 atm.) une solution de 960 mg du produit obtenu au stade F dans 10 ml de méthanol en présence de 150 mg de palladium sur charbon. Après filtration, on évapore à sec sous pression réduite et chromatographie le résidu sur silice (éluant acétate d'éthyle-cyclohexane (4-6). On recueille 759 mg de produit recherché.
RMN CDCl₃ 200 MHz
1,52 (m) : OH ; 3,74 (m) - 1,97 (m) - 2,92 (dt) : les CH₂ ; 7,40 à 7,90 (m) : 5H aromatiques ; 7,53 (t,J = 1) : H thiazole.

### Stade H : 2-phényl 5-thiazole propanal

A une solution refroidie à 10°C de 584 mg du produit obtenu au stade précédent, 800 µl de diméthylsulfoxyde, 1,15 ml de triéthylamine et 8 ml de chlorure de méthylène, on ajoute en maintenant la température à 10°C, 1,27 g de complexe pyridinium sulfotrioxyde, on agite 1 h 15 à 10°C, puis laisse revenir à la température ambiante, on extrait avec du chlorure de méthylène, lave à l'eau, sèche et évapore à sec sous pression réduite, on recueille 806 mg de produit que l'on chromatographie sur silice (éluant acétate d'éthyle-cyclohexane (3-7)), on obtient 450 mg du produit recherché.
RMN CDCl₃ 200 MHz
2,88-3,20 (t) : les CH₂ propyle ; 7,55 (s) : H thiazole ; 7,40 (m) : 3H et 7,87 (m) 2H : les H aromatiques ; 9,85 (sl) : CHO.

### EXEMPLE 8 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl) (2-(3-(4-phényl 1H-imidazol 1-yl) propyl) hydrazono)) érythromycine

On agite pendant 20 heures 125 mg du produit obtenu à l'exemple 1, 80 mg de 3-(4-phényl 1H-imidazole 1-yl) propanal (dont la préparation est donnée ci-après) et 2 ml de méthanol. On ajoute 54 mg de cyanoborohydrure de sodium. On concentre sous pression réduite, reprend avec 20 ml d'acétate d'éthyle, lave avec de la soude puis avec de l'eau saturée de chlorure de sodium, sèche et évapore à sec sous pression réduite, et chromatographie le résidu sur silice (éluant chloroforme-méthanol-ammoniaque 95/5/0,5) on reprend le produit brut par un mélange éther-acétate d'éthyle, filtre, évapore à sec et recueille 85 mg du produit recherché.

| Analyse pour C₄₃H₆₅N₅O₁₀ 812,02 | | | |
|---|---|---|---|
| | C | H | N |
| % calculés | 63,6 | 8,07 | 8,62 |
| % trouvés | 63,4 | 8,2 | 8,3 |

RMN CDCl₃ 400 MHz
3,70 (s) : : H en 11 ; 4,98 (dd) : H₁₃ ; 3,86 (q) : H en 2 ; 2,26 (s) : N-(CH₃)₂ ; 2,63 : 6-OCH₃ ; 5,54 (t) : NH ; 4,27 et 1,97 : les CH₂ propyle ; 7,3 (d)- 7,57 (d) : 2H imidazole ; 7,2 - 7,35 - 7,8 : les aromatiques.

### PREPARATION DE L'EXEMPLE 8 : 3-(4-phényl 1H-imidazol 1-yl) propanal.

### Stade A : 3-(4-phényl 1H-imidazol 1-yl) éthyl 1,3-dioxolane.

On opère comme au stade A de la préparation de l'exemple 6, en utilisant au départ 1,44 g de 4-phénylimidazole et 1,17 ml de bromoéthyldioxolane, on obtient après chromatographie sur silice (éluant ACOEt) 1,8 g de produit attendu.
RMN CDCl₃ 2,19 (d,t) et 4,13 (t) : CH₂ propyl ; 3,8-4,05 : les CH₂ du dioxolane ; 4,88 (t) : H oxolane ; 7,23 et 7,53 : les CH imidazole ; 7,23 - 7,37 - 7,75 : les aromatiques.

### Stade B : 3-(4-phényl 1H-imidazol 1-yl) propanal

On chauffe 20 h à 60°C 1,77 g de produit obtenu au stade A ci-dessus, 35 ml d'acétone et 30 ml d'acide chlorhydrique 2N. On élimine ensuite l'acétone sous pression réduite et neutralise la solution en ajoutant du bicarbonate de sodium en branches, puis on extrait avec de l'acétate d'éthyle, sèche et évapore à sec sous pression réduite. On chromatographie le résidu sur silice (éluant acétate d'éthyle-méthanol (97-3)). On recueille 900 mg de produit recherché.
RMN CDCl₃ 250 MHz
9,81 (s) : CHO ; 7,10 à 7,76 : les H imidazole et aromatiques ; 3,01 (t) et 4,29 (t) : les H propyle.

### EXEMPLE 9 : 11,12-didéoxy 3-de((2,6-didéoxy 3-C-méthyl 3-O-méthyl alpha-L-ribohexopyranosyl) oxy) 6-O-méthyl 12,11-(oxycarbonyl (2-(3-(3-phényl 1,2,4-oxodiazol 5-yl) propyl) hydrazono) érythromycine

On opère comme à l'exemple 6 à partir de 125 mg du produit obtenu comme à l'exemple 1, en utilisant 40 mg de 3-(3-phényl 1,2,4-oxadiazol 5-yl) propanal (dont la préparation est décrite ci-après). Après chromatographie sur silice, éluant éther isopropylique-triéthylamine-méthanol (90-10-10) et cristallisation dans l'éther isopropylique-méthanol, on obtient 107 mg de produit attendu.

| Analyse pour C₄₂H₆₃N₅O₁₁ 814,00 | | | |
|---|---|---|---|
| | C | H | N |
| % calculés | 61,97 | 7,8 | 8,6 |
| % trouvés | 61,7 | 7,9 | 8,5 |

RMN CDCl₃ 300 MHz
3,74 (s) : H₁₁ ; 5,03 (dd) : H₁₃ ; 3,87 (q) : H₂ ; 2,27 (s) : 6-OCH₃ ; 2,27 (s) : N-(CH₃)₂ ; 5,49 (t) : NH ; 3,17 (m) et 2,11 (m) : les CH₂ propyle ; 7,47 à 8,08 : les aromatiques.

### PREPARATION DE L'EXEMPLE 9 : 3-(3-phényl 1,2,4-oxadiazol 5-yl) propanal

### Stade A : 3-(3-phényl 1,2,4-oxadiazol 5-yl) propanol

On agite 1 h, à température ambiante, une solution de 2,5 ml de complexe borane-méthyl sulfure, en solution 2M dans le tétrahydrofurane, 920 mg d'acide 3-(3-phényl 1,2,4-oxadiazol 5-yl) propanoïque (préparé selon R.M. SRIRASTAVA et al. J. Heterocycl. Chem., 21, 1193 (1984) et 20 ml de tétrahydrofuranne. On ajoute en 5 minutes 10 ml de méthanol. On évapore à sec sous pression réduite et chromatographie le résidu sur silice (éluant acétate d'éthyle-hexane (6-4)). On recueille 485 mg de produit attendu.
RMN CDCl₃ 250 MHz
2,07 (si) : OH ; 2,14 (m) - 3,10 (t) - 3,8 (t) : les CH₂ ; 7,41 - 7,54 - 8,06 : les aromatiques.

### Stade B : 3-(3-phényl 1,2,4-oxadiazol 5-yl) propanal

A une solution refroidie à 10°C de 460 mg du produit obtenu au stade A, 680 µl de diméthyl sulfoxyde et 970 µl de triéthylamine dans 5 ml de chlorure de méthylène, on ajoute, en maintenant à 10°C, 1,07 g de complexe pyridinium sulfotrioxyde, on laisse revenir à température ambiante, ajoute 15 ml de chlorure de méthylène, lave à l'eau, sèche, évapore à sec sous pression réduite et chromatographie sur silice (éluant acétate d'éthyle-hexane 4-6) on obtient 365 mg du produit recherché.
RMN CDCl₃
3,13 (m) - 3,26 (m) : les CH₂ ; 7,49 à 8,05 : les aromatiques.

### EXEMPLE 10 : 11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (2-(3-(2-chlorophényl) propyl) hydrazono) érythromycine

On opère comme à l'exemple 6 à partir de 125 mg du produit obtenu à l'exemple 1, en utilisant 67 mg de 2-chlorophényl propanal (dont la préparation est donnée ci-après). Après chromatographie sur silice (éluant éther isopropylique-triéthylamine-méthanol (90-10-10)), on recueille 48 mg de produit recherché.

| Analyse pour C₄₀H₆₂ClN₃O₁₀ 780,40 | | | | |
|---|---|---|---|---|
| | C | H | N | Cl |
| % calculés | 61,56 | 8,01 | 5,38 | 4,45 |
| % trouvés | 61,4 | 8,0 | 5,4 | 4,5 |

RMN CDCl₃ 400 MHz
3,73 (s) : H en 11 ; 5,13 (dd) : H en 13 ; 3,87 (q) : H en 2 ; 2,26 (s) : N-(CH₃)₂ ; 2,64 (s) : 6-OCH₃ ; 5,36 (t) : NH ; 1,83 (m) - 2,70 (m) - 2,79 (m) : les CH₂ ; 7,05 à 7,2 : les aromatiques.

### PREPARATION DE L'EXEMPLE 10 : 3-(2-chlorophényl) propanal

### Stade A : 3-(2-chlorophényl propanoate de méthyle

On agite pendant 1 heure sous atmosphère inerte 4,35 g d'acide métachlorocinamique, 430 mg de palladium sur charbon actif et 70 ml de méthanol. On agite ensuite pendant 3 heures sous atmosphère d'hydrogène. On filtre et évapore à sec sous pression réduite et chromatographie le résidu sur silice (éluant acétate d'éthyle-hexane (2-8)), on obtient 3,1 g de produit recherché.
RMN CDCl₃ 250 MHz
2,6 (t) - 2,8 (t) : les CH₂ ; 3,6 (s) : OCH₃ ; 7,05 - 7,37 : les aromatiques.

### Stade B : 3-(2-chlorophényl) propanol

A une solution de 1,85 g du produit obtenu au stade A, dans 20 ml de tétrahydrofuranne, on ajoute à 0°C, 30 ml d'hydrure de diisobutylaluminium en solution 1M dans le tétrahydrofuranne. On laisse revenir à tempétrature ambiante et agite 2 heures. On ajoute une solution de tartrate mixte sodium-potassium, dilue avec du tétrahydrofuranne, filtre et évapore à sec sous pression réduite. On chromatographie le résidu sur silice (éluant acétate d'éthyle-hexane (2-8)) on obtient 1 g de produit recherché.

### Stade C : 3-(2-chlorophényl) propanal

On opère comme au stade B de la préparation 9, en partant de 1 g du produit obtenu au stade B ci-dessus, en utilisant 2,5 ml de triéthylamine, 1,75 ml de diméthyl sulfoxyde et 2,8 g de complexe pyridinium sulfotrioxyde. Après chromatographie sur silice éluant acétate d'éthyle-hexane (1-9), on obtient 425 mg (43 %) de produit recherché.
RMN CDCl₃ 250 MHz
2,79 (m) et 2,94 (m) : les CH₂ ; 7,05 à 7,25 : les aromatiques ; 9,82 (t) : CHO.

### EXEMPLE 11 : 11,12-didéoxy 3-de((2,6-didéoxy 3-C-méthyl 3-O-méthyl alpha-L-ribohexopyranosyl) oxy) 6-O-méthyl 3-oxo 12,11(oxycarbonyl (2-(3-(4-quinoléinyl) 2-propyl) hydrazono)) érythromycine

### Stade A : 11,12-didéoxy 3-de-((2,6-didéoxy 3-C-méthyl 3-O-méthyl alpha L-ribohexopyranosyl) oxy) 6-O-méthyl 3-oxo 12,11-(oxycarbonyl (hydrazono) érythromycine isomère 10(R) et isomère 10(S) correspondant.

On met en solution dans 176 ml de cyanure de méthyle 17,65 g de 2'-acétate de 11-déoxy 10,11-didéhydro 3-de(2,6-didéoxy 3-C-méthyl 3-O-méthyl alpha-L-ribohexopyranosyl) oxy) 12-O-((1H-imidazol-1-yl) carbonyl) 6-O-méthyl 3-oxo érythromycine. On ajoute 4,07 g de carbonate de césium et 25,5 ml d'hydrate d'hydrazine. On chauffe 10 minutes à 85°C, chasse le solvant sous pression réduite à 40°C, extrait au chlorure de méthylène, lave à l'eau, sèche, évapore le solvant, reprend le résidu dans le méthanol, essore le précipité, le sèche à 50°C sous pression réduite et recueille 6,04 g de produit. On concentre à sec les liqueurs mères, chromatographie sur silice le résidu (éluant : éther isopropylique-méthanol-triéthylamine 80-10-10) et récupère 0,83 g d'isomère A (rf = 0,4) et 2,65 g d'isomère B (rf = 0,2).

### Stade B : 11,12-didéoxy 3-de((2,6-didéoxy 3-C-méthyl 3-O-méthyl alpha-L-ribohexopyranosyl) oxy) 6-O-méthyl 3-oxo 12,11(oxycarbonyl (2-(3-(4-quinoléinyl) 2-propyl) hydrazono)) érythromycine.

On met en suspension dans 130 ml de méthanol 13 g de produit obtenu comme au stade A et 4,66 g de 4-quinoléine propanal préparé comme indiqué ci-dessous. On ajoute 4,8 ml d'acide acétique et agite 20 heures à température ambiante. On ajoute ensuite 5,3 g de cyanoborohydrure de sodium puis poursuit l'agitation pendant 4 heures. On élimine le méthanol sous pression réduite, extrait à l'acétate d'éthyle, lave avec une solution aqueuse de soude N puis à l'eau ; on évapore le solvant de la phase organique, chromatographie le résidu sur silice (éluant : acétate d'éthyle-triéthylamine 97-3) et recueille 12,7 g de produit rf = 0,15. Après une nouvelle chromatographie sur silice (éluant : chlorure de méthylène-méthanol 95-5 puis 85-15) et une cristallisation dans l'éther isopropylique, on obtient le produit pur F = 183°C, dont les analyses sont identiques à celles de l'exemple 5.

### PREPARATION DE L'EXEMPLE 11 : 4-quinoléine propanal.

### Stade A : 2-(4-quinoléinyl éthényl) 1,3-dioxolane.

On met en suspension dans 40 ml de tétrahydrofuranne 3,15 g de 4-quinoléine carboxaldéhyde et 8,6 g de bromure de [1,3-(dioxalan-2-yl) méthyl] triphénylphosphonium, refroidit à -30°C puis ajoute 2,5 g de terbutylate de potassium et agite 1 heure. On laisse revenir à température ambiante, agite 3 heures, verse dans un mélange eau/glace, extrait au chlorure de méthylène, lave à l'eau, sèche, évapore le solvant sous pression réduite, reprend dans un mélange éther éthylique-pentane 3-7, agite 2 heures, filtre et évapore le solvant du filtrat et obtient 3,99 g de produit attendu.

### Stade B : 2-[2-(4-quinoléinyl) éthyl] 1,3-dioxolane.

On dissout dans 40 ml de méthanol 4,3 g de produit obtenu au stade A, ajoute 0,215 g de charbon actif à 10% de palladium et hydogène pendant 2 heures sous une pression de 1500 mbars. On filtre, rince au méthanol, évapore le solvant et recueille 4,2 g de produit attendu utilisé tel quel au stade suivant.

### Stade C : 4-quinoléine propanal.

On dissout 4,2 g du produit obtenu au stade B dans 70 ml d'acétone et ajoute 70 ml d'acide chlorhydrique 2N. On chauffe 6 heures à 40°C, élimine l'acétone sous pression réduite, extrait à l'acétate d'éthyle, lave à l'eau, amène la phase aqueuse à pH = 9 avec une solution aqueuse d'ammoniaque. On extrait à l'acétate d'éthyle, réunit les phases organiques, les sèche et évapore le solvant. On obtient après chromatographie sur silice (éluant : acétate d'éthyle-cyclohexane 6-4) 1,36 g de produit attendu.

### EXEMPLE 12 : 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(7-méthoxy-4-quinoléinyl) propyl) hydrazono)) érythromycine.

On dissout dans 2 ml de méthanol 299 mg de 7-méthoxy 4-quinoléine propanal préparé comme indiqué ci-dessous et 313,9 mg de produit A préparé à l'exemple 1 et 120 µl d'acide acétique. On agite 2 heures 15 minutes à température ambiante puis ajoute 62,84 mg de cyanoborohydrure de sodium. On agite 20 heures à température ambiante. On verse le milieu réactionnel sur 50 ml d'acétate d'éthyle, lave avec 15 ml de soude N puis à l'eau, sèche, évapore le solvant sous pression réduite et recueille 549 mg de produit que l'on purifie par chromatographie sur silice (éluant : éther ispropylique-méthanol-triéthylamine 80-10-10) puis (chloroforme-méthanol-ammoniaque 96-4-0,4). On récupère 37, 2 mg de produit attendu rf = 0,2.

| Analyse | | | |
|---|---|---|---|
| | C | H | N |
| % calculés | 63,84 | 8,04 | 6,77 |
| % trouvés | 63,8 | 8,1 | 6,6 |

RMN CDCl₃ 300 MHz
3,74 (s) : H₁₁ ; 3,17 (m) : NH-CH₂ ; 3,95 (s) : OCH₃ de la quinoléine ; 7,16-7,41 (d)-8,00 (d)-8,70 (d) : : H quinoléine ; 3,87 (q) : H₂ ; 2,65 (s) : 6-OCH₃ ; 2,65 (m) : H₈ ; 0,82 (t) : CH₃-CH₂.

### PREPARATION DE L'EXEMPLE 12 : 7-méthoxy 4-quinoléine propanal.

### Stade A : 2-[(7-méthoxy 4-quinoléinyl) éthényl] 1,3-dioxolane.

On opère comme à la préparation de l'exemple 11, stade A en utilisant au départ 787 mg de 7-méthoxy 4-quinoléine carboxaldéhyde. On obtient 2,61 g de produit que l'on chromatographie sur silice (éluant : chloroforme-acétate d'éthyle 7-3). On obtient 931 mg de produit attendu.

### Stade B : 2-[2-(7-méthoxy 4-quinoléinyl) éthyl] 1,3-dioxolane.

On opère comme à la préparation de l'exemple 11 stade B en utilisant 931 mg de produit préparé au stade A et obtient 869 mg de produit attendu.

### Stade C : 2-(7-méthoxy 4-quinoléine) propanal.

On opère comme à la préparation de l'exemple 11 stade C en utilisant 845 mg de produit obtenu au stade B. On obtient 310 mg de produit attendu rf = 0,15.

### EXEMPLE 13 : 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(2-(3-pyridinyl-4-thiazolyl) propyl) hydrazono)) érythromycine.

On opère comme à l'exemple 12, en utilisant au départ dans 3,7 ml de méthanol, 158 mg de 2-(3-pyridinyl) 4-thiazole propanal, 370 mg de produit A préparé à l'exemple 1 et 70 µl d'acide acétique puis après 4 heures d'agitation à température ambiante 75 mg de cyanoborohydrure de sodium. Après 16 heures d'agitation à température ambiante, on ajoute de nouveau 16 mg d'aldéhyde et 20 mg de réactif réducteur et poursuit l'agitation pendant 3 heures. On ajoute de l'eau, de l'acétate d'éthyle, alcalinise à pH = 9 à l'aide d'ammoniaque, lave la phase organique à l'eau, la sèche et évapore le solvant sous pression réduite. Après chromatographie sur silice (éluant : éther isopropylique-méthanol-triéthylamine 80-10-10), on obtient 203 mg de produit attendu.
3,18 (m) : H₁₀ ; 3,74 (s) : H₁₁ ; 7,05 (s) : H₅ thiazole ; 7,37 (dd)-8,24 (ddd)-8,62 (dd)-9,13 (dd) : pyridine ; 3,86 (q) : H₂ ; 2,65 (s) : 6-OCH₃ ; 2,66 (m) : H₈ ; 0,85 (t) : CH₃-CH₂.

### PREPARATION DE L'EXEMPLE 13 : 2-(3-pyridinyl) 4-thiazole propanal.

### Stade A : [[2-(3-pyridinyl) 4-thiazolyl] éthényl] 1,3-dioxolane.

On opère comme à la préparation de l'exemple 11 stade A en utilisant au départ 2,6 g de 2-(3-pyridinyl) 4-thiazolyl carboxaldéhyde. On obtient après chromatographie sur silice (éluant : acétate d'éthyle-hexane 2-1) 4,8 g de produit attendu (rf = 0,35) utilisé tel quel pour le stade suivant.

### Stade B : 2-[2-((3-pyridinyl) 4-thiazolyl) éthyl] 1,3-dioxolane.

On opère comme à la préparation de l'exemple 11 stade B en utilisant au départ 4,8 g de produit préparé au stade A et obtient après chromatographie du résidu sur silice (éluant : acétate d'éthyle-cyclohexane 2-1) 1,4 g de produit attendu.

### Stade C : 2-(3-pyridinyl) 4-thiazolyl propanal.

On opère comme à la préparation de l'exemple 11 stade C en utilisant au départ 1,2 g de produit préparé au stade B. On obtient après chromatographie sur silice (éluant : acétate d'éthyle-hexane 2-1) 468 mg de produit attendu.

En opérant comme dans les exemples précédents en utilisant au départ le composé de l'exemple 1 et l'aldéhyde approprié, on a préparé les produits suivants :

### EXEMPLE 14 : 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(1H-imidazol-1-yl) propyl) hydrazono)) érythromycine.

RMN (CDCl₃) 300 MHz :
0,83 (t) : CH₃-CH₂ ; 1,08 (d)-1,17 (d)-1,25 (d)-1,3 (d)-1,35 (d) : les CH₃-CH ; 1,3 (s)-1,47 (s) : 6 et 12 Me ; 2,12 (m) : CH₂-CH₂-CH₂ ; 2,27 (s) : N(Me)₂ ; 2,45 (m) : H'₃ ; 2,59 (s) : 6-OMe ; 3,05 (m) : H₄ ; 2,6 à 3,2 : H'₂, H₁₀ : H₈ et CH₂NH ; 3,53 (m) : H'₅ ; 3,72 (s) : H₁₁ ; 3,85 (q) : H₂ ; 4,27 : H'₁ et H₅ ; 4,63 (m) : CH₂-N ; 4,99 (dd) : H₁₃ ; 5,46 (t) : NH-CH₂ ; 7,10-7,64-7,66-7,97 : aromatiques.

### EXEMPLE 15 : 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(3H-imidazo (4,5-b)pyridin-3-yl) propyl) hydrazono)) érythromycine.

RMN (CDCl₃) 300 MHz :
0,85 (t) : CH₃-CH₂ ; 1,09-1,19 (d)-1,25 (d)-1,31 (d)-1,34 (d): les CH₃CH ; 1,33 et 1,48 : 6 et 12 Me ; 1,57 et 1,96 : CH₂ en 14;
1,66 et 1,87 : CH₂ en 7 ; 2,05 et 2,18 : CH₂-CH₂-CH₂ ; 2,26 (s) : N(CH₃)₂ ; 2,44 (m) : H'₃ ; 2,6 (s) : 6-OCH₃ ; 2,66 (m) : H₈ ; 2,70 à 2,85 : CH₂NH ; 3,04 (m) : H₄ ; 3,18 : H₂, H₁₀ ; 3,70 (s) : H₁₁ ; 3,85 (q) : H₂ ; 4,27 : H'₁ et H₅ ; 4,42 à 4,70 : CH₂-N ; 4,97 (dd) : H₁₃ ; 5,56 (t) : NH ; 8,22 (dd)-8,05 (d)-8,28 (s)-8,38 (d) : aromatiques.

### EXEMPLE 16 : 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(1,1'-biphényl-4-yl) propyl) hydrazono)) érythromycine.

RMN (CDCl₃) 300 MHz :
0,87 (t) : CH₃-CH₂ ; 1,08 (d)-1,18 (d)-1,23 (d)-1,32 (d)-1,38 (d) : les CH₃-CH ; 1,34 (s)et 1,48 (s) : 6 et 12 Me ; 2,26 (s) : N(CH₃)₂ ; 2,44 (m) : H'₃ ; 2,65 (s) : 6-OCH₃ ; 2,65 (m) H₈ ; 2,77 (m) CH₂-Ar ; 2,85 (t) : CH₂NH ; 3,07 (m) : H₄ ; 3,18 (m) : H'₂, H₁₀ ; 3,25 (m) : H'₅ ; 3,76 (s) : H₁₁ ; 3,87 (q) : H₂ ; 4,27 : H'₁ et H₅ ; 5,04 (dd) : H₁₃ ; 5,37 (t) : NH-CH₂ ; 7,25 à 7,6 : aromatiques.

### EXEMPLE 17 : 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(2-phényl 4-thiazolyl) propyl) hydrazono)) érythromycine.

RMN (CDCl₃) 300 MHz :
0,86 (t) : CH₃-CH₂ ; 1,07 (d)-1,19 (d)-1,24 (d)-1,31 (d)-1,35 (d) : les CH₃-CH ; 1,32 (s)-1,48 (s) : 6-CH₃ et 12-CH₃ ; 2,26 (s) : N(CH₃)₂ ; 2,65 (s) : 6-OCH₃ : 2,45 (m) : H'₃ ; 2,65 (m) : H₈ ; 2,8 à 3,25 (m) : H₄, H₁₀, H'₂, CH₂-Ar et CH₂N; 3,53 (m) : H'₅ ; 3,76 (m) : H₁₁ ; 3,86 (q) : H₂ ; 4,27 (d) : H'₁ et H₅ ; 5,04 (dd) : H₁₃ ; 5,36 (t) : NH ; 6,96-7,40-7,93 : aromatiques.

### EXEMPLE 18 : 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(5-phényl 1,2,4-thiadiazol-3-yl) propyl) hydrazono)) érythromycine.

RMN (CDCl₃) 300 MHz :
0,87 (t) : CH₃-CH₂ ; 1,33 et 1,47 : 6 et 12 Me ; 2,17 (m) : CH₂-CH₂-CH₂ ; 2,26 (s) : N(CH₃)₂ ; 2,67 (s) : 6-OCH₃ ; 2,67 (s) : H₈ ; 3,76 (s) : H₁₁ ; 3,85 (q) : H₂ ; 5,06 (dd) : H₁₃ ; 5,39 (t) : NH-CH₂ ; 7,49-7,94 : aromatiques.

### EXEMPLE 19 : 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(4-(4-chlorophényl-1H-imidazol-1-yl) propyl) hydrazono)) érythromycine.

### EXEMPLE 20 : 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(6-méthoxy-4-quinoléinyl) propyl) hydrazono)) érythromycine.

En opérant comme à l'exemple 12, on a obtenu le produit recherché.
RMN CDCl₃ 300 MHz
3,74 (s) : H₁₁ ; 5,52 (tl) : NH-CH₂ ; 3,98 (s) : OCH₃ de la quinoléine ; 7,25-7,35 (d)-7,99 (d)-8,65 (d) : H quinoléine ; 3,87 (q) : H₂ ; 2,64 (s) : 6-OCH₃ ; 2,64 (m) : H₈ ; 5,02 (dd) H₁₃.

En opérant comme précédemment, on a préparé les composés de formule (I) dans lesquels le radical 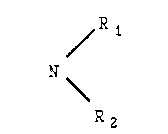 représente le radical

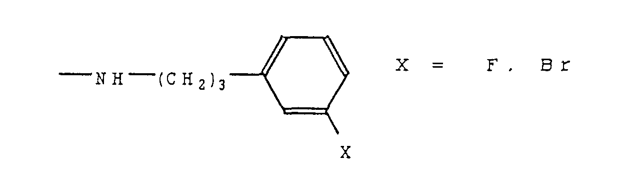

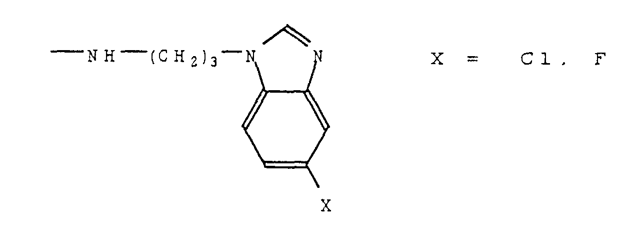

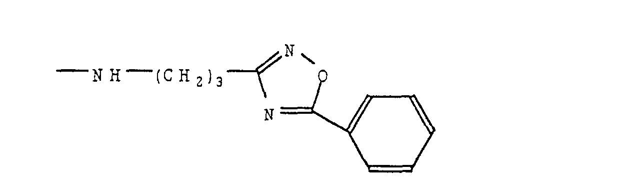

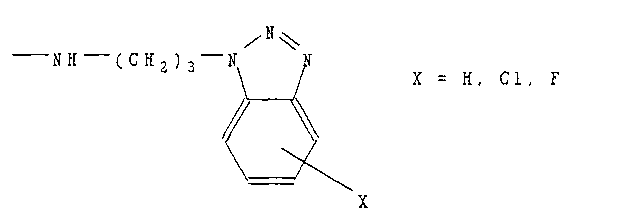

### EXEMPLE DE COMPOSITION PHARMACEUTIQUE

On a préparé des comprimés renfermant :

| | |
|---|---|
| Produit de l'exemple 5 | 150 mg |
| Excipient q.s.p. | 1 g |

Détail de l'excipient amidon, talc,
stéarate de magnésium

### ETUDE PHARMACOLOGIQUE DES PRODUITS DE L'INVENTION

### Méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne.

Après incubation de vingt-quatre heures à l'étuve à 37°C, l'inhibition de la croissance est appréciée par transillumination, ce qui permet de déterminer les concentrations minimales inhibitrices (C.M.I.) exprimées en microgrammes/cm³.

Les résultats suivants ont été obtenus avec le produit de l'exemple 5 : (lecture après 24 heures)

| Souches bactériennes à GRAM⁺ | |
|---|---|
| Staphylococcus aureus 011UC4 | 0,02 |
| Staphylococcus aureus 011G025I | 0,08 |
| Staphylococcus epidermidis 012G011I | 0,04 |
| Streptococcus pyogenes groupe A 02A1UC1 | ≤ 0,02 |
| Streptococcus agalactiae groupe B 02B1HT1 | ≤ 0,02 |
| Streptococcus faecalis groupe D 02D2UC1 | ≤ 0,02 |
| Streptococcus faecium groupe D 02D3HT1 | ≤ 0,02 |
| Streptococcus sp groupe G 02G0GR5 | ≤ 0,02 |
| Streptococcus mitis 02mitCB1 | ≤ 0,02 |
| Streptococcus agalactiae groupe B 02B1SJ1 | ≤ 0,02 |
| Streptococcus pneumoniae 032UC1 | ≤ 0,02 |
| Streptococcus pneumoniae 030SJ5 | ≤ 0,02 |

De plus, le produit de l'exemple 5, a manifesté une activité intéressante sur les souches bactériennes à GRAM⁻ Haemophilus Influenzae 351HT3, 351CB12, 351CA1 et 351GR6.

Les produits des exemples 12 et 13 ont manifesté également une excellente activité sur les souches bactériennes à gram (+) et gram (-).

Ainsi, en opérant comme indiqué ci-dessus, les résultats suivants ont été obtenus avec les produits des exemples 12 et 13 (lecture après 24 heures).

| Souches bactériennes à GRAM⁺ | Ex.12 | Ex.13 |
|---|---|---|
| Staphylococcus aureus 011UC4 | 0,08 | 0,04 |
| Staphylococcus aureus 011G025I | 0,08 | 0,15 |
| Staphylococcus epidermidis 012GO11I | 0,04 | 0,04 |
| Streptococcus pyogenes 02A1UC1 | ≤ 0,02 | ≤ 0,02 |
| Streptococcus agalactiae 02B1HT1 | ≤ 0,02 | ≤ 0,02 |
| Streptococcus faecalis 02D2UC1 | ≤ 0,02 | ≤ 0,02 |
| Streptococcus faecium 02D3HT1 | ≤ 0,02 | ≤ 0,02 |
| Streptococcus pneumoniae 032UC1 | 0,04 | ≤ 0,02 |
| Streptococcus pneumoniae 03OSJ5I | ≤ 0,02 | ≤ 0,02 |
| Streptococcus pneumoniae 030CR18C | 0,6 | 0,6 |
| Haemophilus inflienzae 351HT3 | 1,2 | 0,6 |
| Haemophilus inflienzae 351CB12 | 1,2 | 1,2 |

## Revendications

1. Les composés de formule générale (I) : 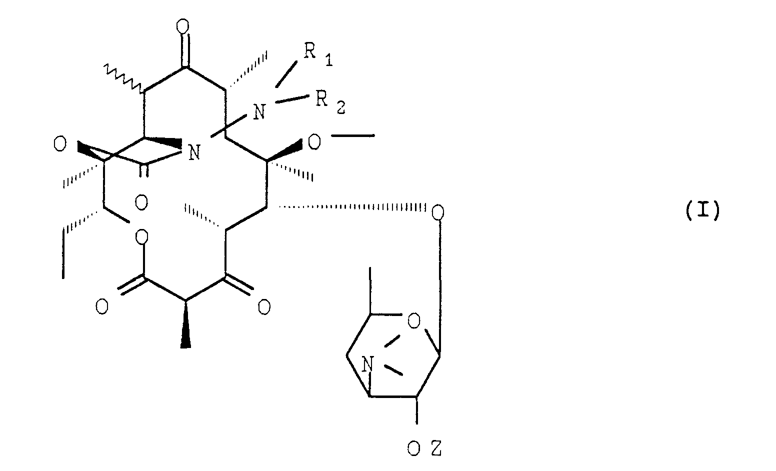 dans lesquels :
ou bien R₁ et R₂ identiques ou différents représentent un atome d'hydrogène, ou un radical hydrocarboné renfermant jusqu'à 24 atomes de carbone, saturé ou insaturé, éventuellement interrompu par un ou plusieurs hétéroatomes et portant éventuellement un ou plusieurs groupements fonctionnels,
ou bien R₁ et R₂ forment avec l'atome d'azote auquel ils sont liés un hétérocycle renfermant éventuellement un ou plusieurs hétéroatomes choisis parmi l'azote, l'oxygène et le soufre,
ou bien R₁ et R₂ forment ensemble un radical 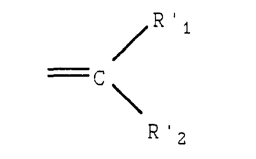 dans lesquels R'₁ et R'₂ identiques ou différents représentent un atome d'hydrogène ou un radical hydrocarboné renfermant jusqu'à 23 atomes de carbone, saturé ou insaturé, éventuellement interrompu par un ou plusieurs hétéroatomes et portant éventuellement un ou plusieurs groupements fonctionnels et Z représente un atome d'hydrogène ou le reste d'un acide carboxylique renfermant jusqu'à 18 atomes de carbone, le trait ondulé en position 10 indiquant que le méthyl peut être de configuration R ou S, ou un mélange de configuration R et S, ainsi que les sels d'addition avec les acides des composés de formule (I).

2. Les composés de formule (I) tels que définis à la revendication 1 dans lesquels Z représente un atome d'hydrogène.

3. Les composés de formule (I) tels que définis à la revendication 1 ou 2 dans lesquels R₁ représente un atome d'hydrogène.

4. Les composés de formule (I) tels que définis à la revendication 1 ou 2 dans lesquels R₁ et R₂ représentent chacun un atome d'hydrogène.

5. Les composés de formule (I) tels que définis à la revendication 1 ou 2 dans lesquels R'₁ représente un atome d'hydrogène.

6. Les composés de formule (I) tels que définis à la revendication 1, 2 ou 5 dans lesquels R₁ et R₂ forment ensemble un radical :
=CH(CH₂)ₙAr₁
dans lequel Ar₁ représente un radical aryle ou hétéroaryle éventuellement substitué et n représente un nombre entier pouvant varier de 0 à 8.

7. Les composés de formule (I) tels que définis à la revendication 1, 2 ou 5 dans lesquels R₁ et R₂ forment ensemble un radical : 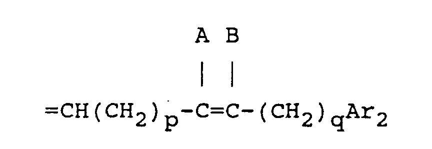 dans lequel p et q identiques ou différents, représentent un nombre entier variant de 0 à 6, A et B identiques ou différents représentent un atome d'hydrogène ou d'halogène ou un radical alkyle renfermant jusqu'à 8 atomes de carbone, la géométrie de la double liaison étant E ou Z ou un mélange E + Z,
ou bien A et B forment une troisième liaison avec les atomes de carbone auxquels ils sont liés et Ar₂ représente un radical aryle ou hétéroaryle, mono ou polycyclique, éventuellement substitué.

8. Les composés de formule (I) tels que définis à la revendication 7, dans lesquels p et q représentent le nombre 0.

9. Les composés de formule (I) tels que définis à la revendication 7 ou 8, dans lesquels A et B représentent un atome d'hydrogène.

10. Les composés de formule (I), tels que définis à la revendication 1, 2 ou 3 dans lesquels R₂ représentent un radical :
(CH₂)ᵣAr₃
dans lesquels r représente un nombre entier variant de 0 à 6 et Ar₃ représente un radical aryle ou hétéroaryle éventuellement substitué.

11. Les composés de formule (I) tels que définis à la revendication 10, dans lesquels Ar₃ représente un radical 4-quinoléinyle éventuellement mono ou polysubstitué sur l'un et/ou l'autre des 2 cycles de la quinoléine.

12. Les composés de formule (I) tels que définis à la revendication 10, dans lesquels Ar₃ représente un radical 4-quinoléinyle non substitué.

13. Les composés de formule (I) tels que définis à la revendication 10, dans lesquels Ar₃ représente un radical 4-quinoléinyle substitué par un radical méthoxy.

14. Les composés de formule(I) tels que définis à la revendication 10 dans lesquels Ar₃ représente un radical thiazolyle substitué par un radical pyridyle.

15. Les composés de formule (I) tels que définis à l'une quelconque des revendications 10 à 14, dans lesquels r représente un nombre entier variant de 1 à 4.

16. Les composés de formule (I) dont les noms suivent :
- 11,12-didéoxy 3-de((2,6-dideoxy 3-C-méthyl 3-O-méthyl alpha L-ribohexopyranosyl) oxy) 6-O-méthyl 3-oxo 12,11-(oxycarbonyl (2-(3-(4-quinoléinyl) 2-propyl) hydrazono)) érythromycine,
- 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(7-méthoxy-4-quinoléinyl) propyl) hydrazono)) érythromycine,
- 11,12-dideoxy 3-de ((2,6-dideoxy 3-C-méthyl 3-O-méthyl alpha-L-ribohexopyranosyl) oxy) 6-O-méthyl 3-oxo 12,11-(oxycarbonyl 2-(3-(2-(3-pyridinyl-4-thiazolyl) propyl) hydrazono)) érythromycine.

17. A titre de médicaments, les composés de formule (I) tels que définis à l'une quelconque des revendications 1 à 15 ainsi que leurs sels d'addition avec les acides pharmaceutiquement acceptables.

18. A titre de médicaments le composé défini à la revendication 16 ainsi que ses sels d'addition avec les acides pharmaceutiquement acceptables.

19. Les compositions pharmaceutiques renfermant comme principe actif au moins un médicament défini à la revendication 17 ou 18.

20. Procédé de préparation des composés de formule (I) tels que définis à la revendication 1, **caractérisé en ce que** l'on soumet un composé de formule (II) : 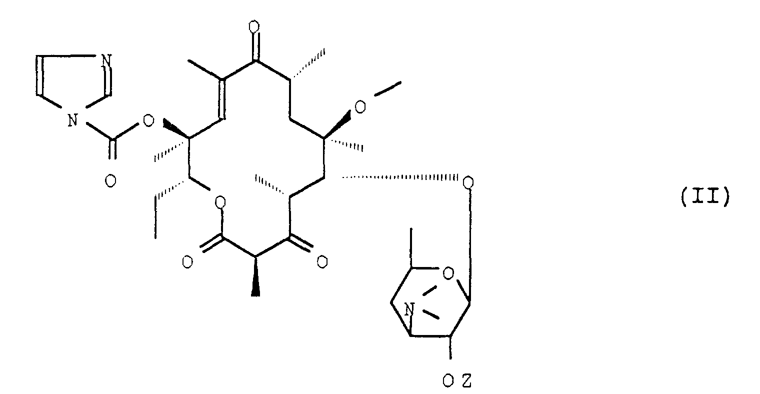 dans laquelle Z conserve la signification indiquée à la revendication 1,
ou bien à l'action de l'hydrazine NH₂NH₂ pour obtenir le composé de formule (I_{A}) : 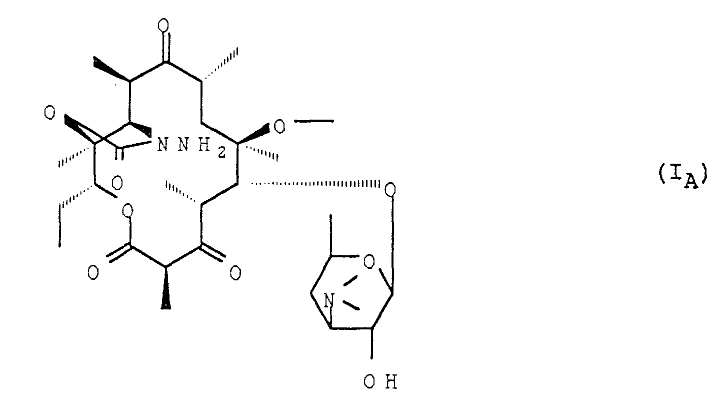 que l'on soumet si désiré à l'action d'un aldéhyde R'₂CHO ou d'une cétone 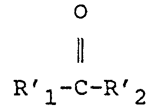 dans lesquels R'₁ et R'₂ ont la signification indiquée à la revendication 1, pour obtenir le composé de formule (I_{B}) correspondant : 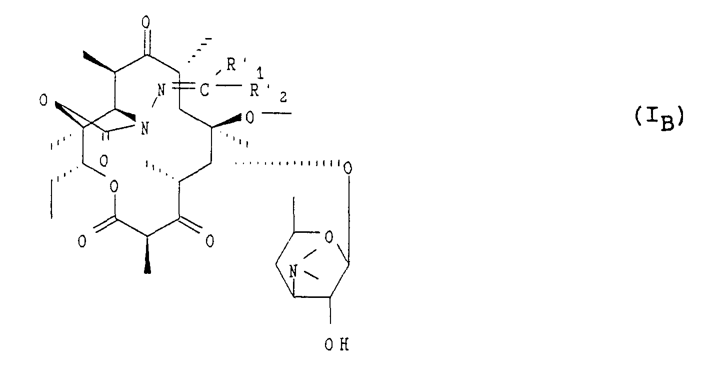 dans laquelle R'₁ et R'₂ conservent la même signification que précédemment, que l'on soumet si désiré à l'action d'un agent de réduction pour obtenir le composé de formule (I_{C}) correspondant : 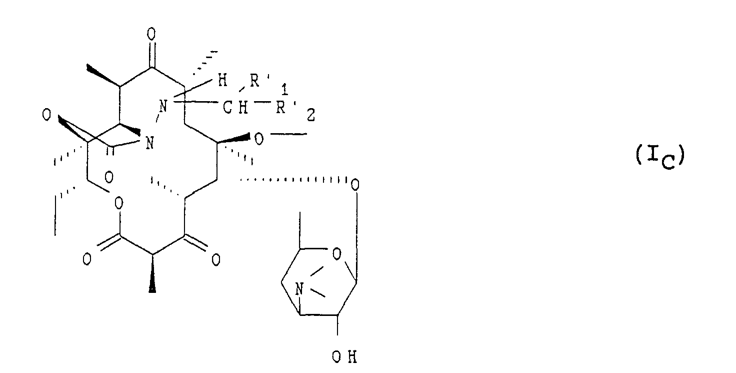 dans laquelle R'₁ et R'₂ conservent la signification précédente, c'est-à-dire un composé de formule (I) dans lequel R₁ représente un atome d'hydrogène et R₂ représente un radical CHR'₁-R'₂, puis si désiré, soumet le composé de formule (I_{C}) à l'action d'un agent susceptible de remplacer l'atome d'hydrogène du groupement NH par un groupement R₁ tel que défini à la revendication 1 à l'exception de la valeur hydrogène, puis si désiré, soumet les composés obtenus à l'action d'un acide pour en former le sel et/ou à l'action d'un agent d'estérification du groupement OH en 2'.

21. Procédé selon la revendication 20, **caractérisé en ce que** l'on soumet le composé de formule (II) : 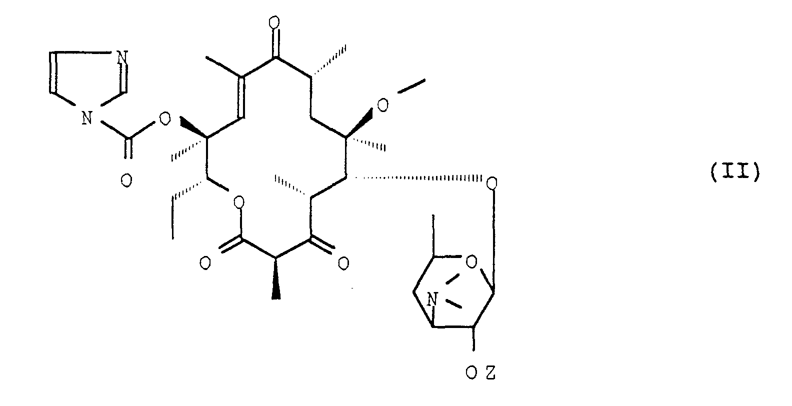 dans lequel Z conserve la signification indiquée à la revendication 1, à l'action d'un composé de formule NH₂NHR₂ dans laquelle R₂ a la signification indiquée à la revendication 1 pour obtenir le composé de formule (I'_{A}) : 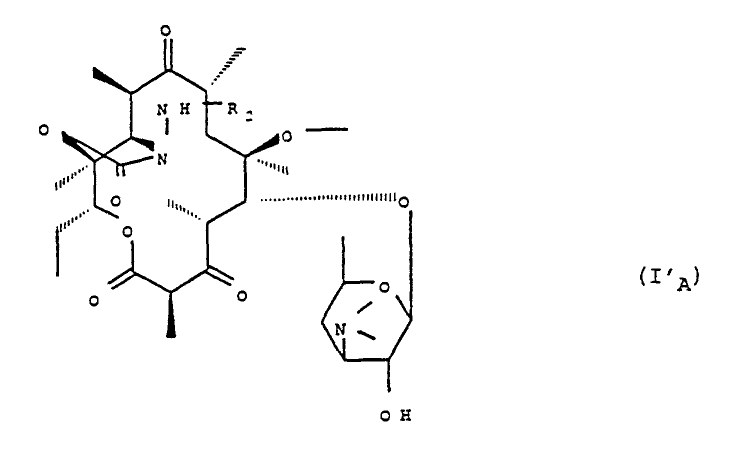 que l'on soumet si désiré, à l'action d'un agent susceptible de remplacer l'atome d'hydrogène du groupement NH par un radical R₁ tel que défini à la revendication 1 à l'exception de la valeur hydrogène pour obtenir le composé de formule (I'_{B}) correspondant : 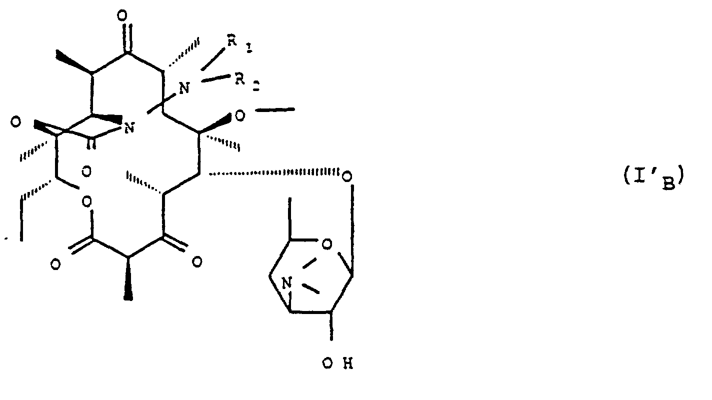 dans laquelle R₁ et R₂ ont la signification indiquée précédemment que l'on soumet si désiré, à l'action d'un agent d'estérification du groupement OH en 2' ou à l'action d'un acide pour en former le sel.

22. A titre de produits chimiques nouveaux, les produits de formule de R'₂CHO ou 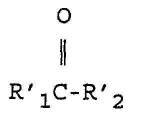 définis à la revendication 20 dont les noms suivent :
- le 2-phényl 5-thiazole propanal
- le 3-(3-phényl-1,2,4-oxadiazol 5-yl)propanal
- le 7-méthoxy 4-quinoléine propanal
- le 2-(3-pyridinyl) 4-thiazole propanal

## Patentansprüche

1. Verbindungen der allgemeinen Formel (I): 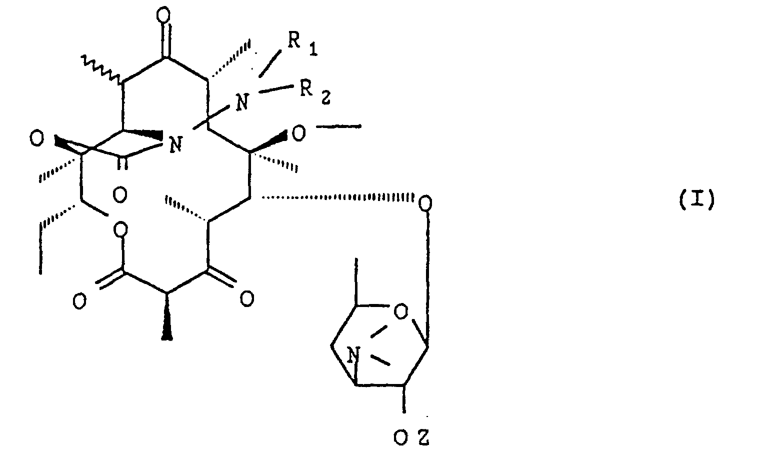 worin:
R₁ und R₂, die gleich oder unterschiedlich sind, ein Wasserstoffatom oder eine gesättigte oder ungesättigte Kohlenwasserstoff-Gruppe, die bis zu 24 Kohlenstoffatome umfaßt und gegebenenfalls durch ein Heteroatom oder mehrere Heteroatome unterbrochen ist und gegebenenfalls eine oder mehrere funktionelle Gruppierung(en) trägt darstellen;
oder R₁ und R₂ mit dem Stickstoffatom, an das sie gebunden sind, einen Heterocyclus bilden, der gegebenenfalls ein Heteroatom oder mehrere Heteroatome, ausgewählt aus Stickstoff, Sauerstoff und Schwefel, umfaßt;
oder R₁ und R₂ zusammen eine Gruppe 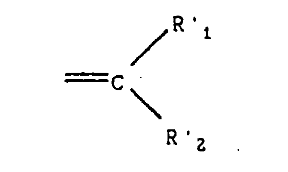 bilden, worin R'₁ und R'₂, die gleich oder unterschiedlich sind, ein Wasserstoffatom oder eine gesättigte oder ungesättigte Kohlenwasserstoff-Gruppe, die bis zu 23 Kohlenstoffatome umfaßt und gegebenenfalls durch ein Heteroatom oder mehrere Heteroatome unterbrochen ist und gegebenenfalls eine oder mehrere funktionelle Gruppierungen trägt, darstellen; und
Z ein Wasserstoffatom oder den Rest einer Carbonsäure, der bis zu 18 Kohlenstoffatome umfaßt, darstellt;
die Wellenlinie in Position 10 anzeigt, daß das Methyl in R- oder S-Konfiguration sein kann oder ein Gemisch aus R- und S-Konfiguration vorliegen kann;
sowie die Additionssalze der Verbindungen der Formel (I) mit Säuren.

2. Verbindungen der Formel (I) nach Anspruch 1, wobei Z ein Wasserstoffatom darstellt.

3. Verbindungen der Formel (I) nach Anspruch 1 oder Anspruch 2, wobei R₁ ein Wasserstoffatom darstellt.

4. Verbindungen der Formel (I) nach Anspruch 1 oder Anspruch 2, wobei R₁ und R₂ jeweils ein Wasserstoffatom darstellen.

5. Verbindungen der Formel (I) nach Anspruch 1 oder 2, wobei R'₁ ein Wasserstoffatom darstellt.

6. Verbindungen der Formel (I) nach Anspruch 1, 2 oder 5, wobei R₁ und R₂ zusammen eine Gruppe:
=CH(CH₂)ₙAr₁
bilden, worin Ar₁ eine Aryl-Gruppe oder Heteroaryl-Gruppe darstellt, die gegebenenfalls substituiert ist, und n eine ganze Zahl zwischen 0 und 8 darstellt.

7. Verbindungen der (I) nach Anspruch 1, 2 oder 5, wobei R₁ und R₂ zusammen eine Gruppe: 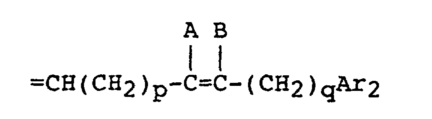 bilden, worin p und q, die gleich oder unterschiedlich sind, eine ganze Zahl zwischen 0 und 6 darstellen, A und B gleich oder unterschiedlich sind und ein Wasserstoffatom oder ein Halogenatom oder eine Alkyl-Gruppe mit bis zu 8 Kohlenstoffatomen darstellen, wobei die Geometrie der Doppelbindung E oder Z oder ein Gemisch aus E und Z ist oder A und B mit den Kohlenstoffatomen, an die sie gebunden sind, eine dritte Bindung bilden und Ar₂ ein Aryl-Gruppe oder Heteroaryl-Gruppe, die mono- oder polycyclisch und gegebenenfalls substituiert ist,
darstellt.

8. Verbindungen der Formel (I) nach Anspruch 7, wobei p und q die Zahl 0 darstellen.

9. Verbindungen der Formel (I) nach Anspruch 7 oder 8, wobei A und B ein Wasserstoffatom darstellen.

10. Verbindungen der Formel (I) nach Anspruch 1, 2 oder 3, wobei R₂ eine Gruppe:
(CH₂)ᵣAr₃
darstellt, worin r eine ganze Zahl zwischen 0 und 6 darstellt und Ar₃ eine Aryl-Gruppe oder Heteroaryl-Gruppe, die gegebenenfalls substituiert ist, darstellt.

11. Verbindungen der Formel (I) nach Anspruch 10, wobei Ar₃ eine 4-Chinolinyl-Gruppe darstellt, die gegebenenfalls an dem einem und/oder dem anderen der zwei Ringe des Chinolins mono- oder polysubstituiert ist.

12. Verbindungen der Formel (I) nach Anspruch 10, wobei Ar₃ eine nicht-substituierte 4-Chinolinyl-Gruppe darstellt.

13. Verbindungen der Formel (I) nach Anspruch 10, wobei Ar₃ eine durch eine Methoxy-Gruppe substituierte 4-Chinolinyl-Gruppe darstellt.

14. Verbindungen der Formel (I) nach Anspruch 10, wobei Ar₃ eine durch eine Pyridyl-Gruppe substituierte Thiazolyl-Gruppe darstellt.

15. Verbindungen der Formel (I) nach einem der Ansprüche 10 bis 14, wobei r eine ganze Zahl zwischen 1 und 4 darstellt.

16. Verbindungen der Formel (I) mit den folgenden Namen:
11,12-Didesoxy-3-de((2,6-didesoxy-3-C-methyl-3-Omethyl-alpha-L-ribohexopyranosyl)oxy)-6-O-methyl-3-oxo-12,11-(oxycarbonyl(2-(3-(4-chinolinyl)2-propyl)hydrazono))erythromycin,
11,12-Didesoxy-3-de((2,6-didesoxy-3-C-methyl-3-Omethyl-alpha-L-ribohexopyranosyl)oxy)-6-O-methyl-3-oxo-12,11-(oxycarbonyl-2-(3-(7-methoxy-4-chinolinyl)propyl)hydrazono))erythromycin,
11,12-Didesoxy-3-de((2,6-didesoxy-3-C-methyl-3-O-methyl-alpha-L-ribohexopyranosyl)oxy)-6-O-methyl-3-oxo-12,11-(oxycarbonyl-2-(3-(2-(3-pyridinyl-4-thiazolyl)propyl)hydrazono))erythromycin.

17. Verbindungen der Formel (I) nach einem der Ansprüche 1 bis 15 sowie ihre Additionssalze mit den pharmazeutisch annehmbaren Säuren als Medikamente.

18. Verbindung nach Anspruch 16 sowie ihre Additionssalze mit den pharmazeutisch annehmbaren Säuren als Medikamente.

19. Pharmazeutische Zusammensetzungen, die als Wirkstoff mindestens ein Medikament, das in Anspruch 17 oder 18 definiert ist, umfassen.

20. Verfahren zur Herstellung der Verbindungen der Formel (I) nach Anspruch 1, **dadurch gekennzeichnet, daß** man eine Verbindung der Formel (II): 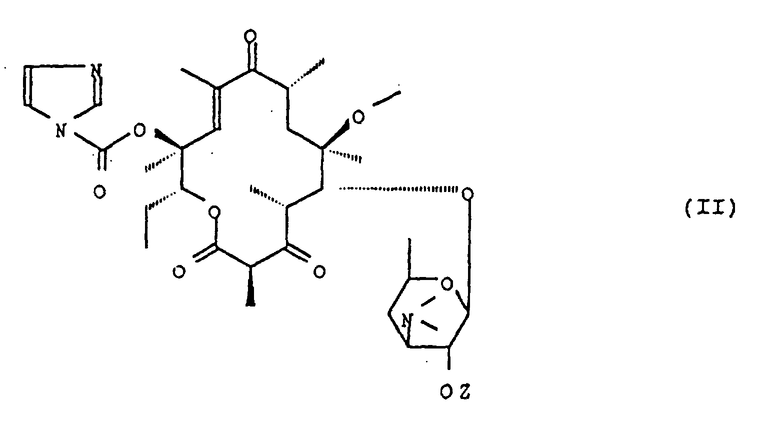 in der Z die in Anspruch 1 gegebene Bedeutung beibehält, der Wirkung von Hydrazin NH₂NH₂ unterwirft, um die Verbindung der Formel (I_{A}) zu erhalten: 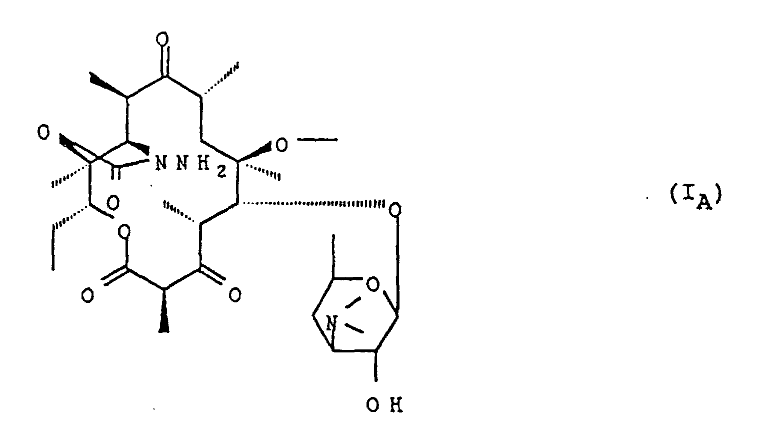 die man, wenn gewünscht, der Wirkung eines Aldehyds R'₂CHO oder eines Ketons 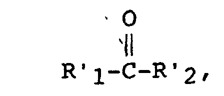 worin R'₁ und R'₂ die in Anspruch 1 gegebene Bedeutung besitzen, unterwirft, um die entsprechende Verbindung der Formel (I_{B}) zu erhalten: 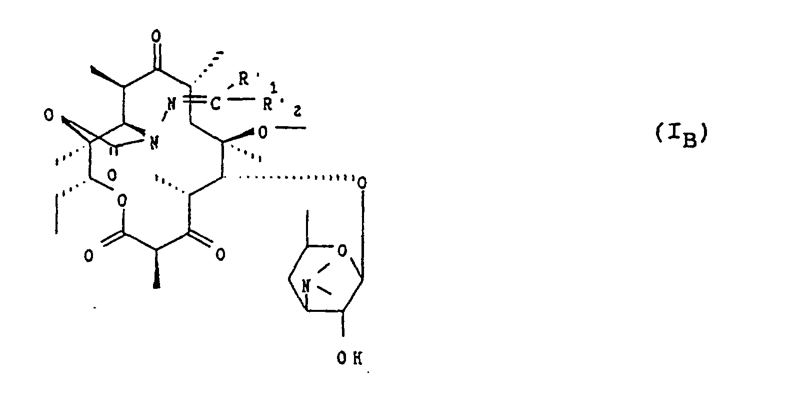 worin R'₁ und R'₂ dieselbe Bedeutung wie vorstehend beibehalten,
die man, wenn gewünscht, der Wirkung eines Reduktionsmittels unterwirft, um die entsprechende Verbindung der Formel (I_{C}) zu erhalten: 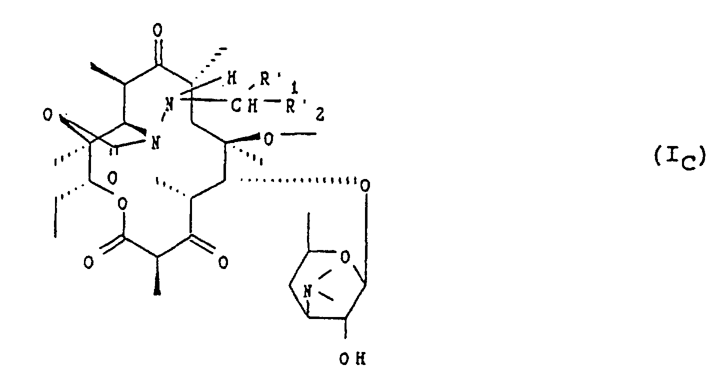 worin R'₁ und R'₂ die vorstehende Bedeutung beibehalten, d.h. eine Verbindung der Formel (I), in der R₁ ein Wasserstoffatom darstellt und R₂ eine Gruppe CHR'₁-R'₂ darstellt; man, wenn gewünscht, die Verbindung der Formel (I_{C}) der Wirkung eines Mittels unterwirft, das geeignet ist, das Wasserstoffatom der Gruppierung NH durch eine Gruppierung R₁, wie sie in Anspruch 1 definiert ist, ausgenommen die Bedeutung Wasserstoff, zu ersetzen, und man dann, wenn gewünscht, die erhaltenen Verbindungen der Wirkung einer Säure, um dadurch ein Salz zu bilden, und/oder der Wirkung eines Veresterungsmittels für die Gruppierung OH in 2' unterwirft.

21. Verfahren nach Anspruch 20, **dadurch gekennzeichnet, daß** man die Verbindung der Formel (II): 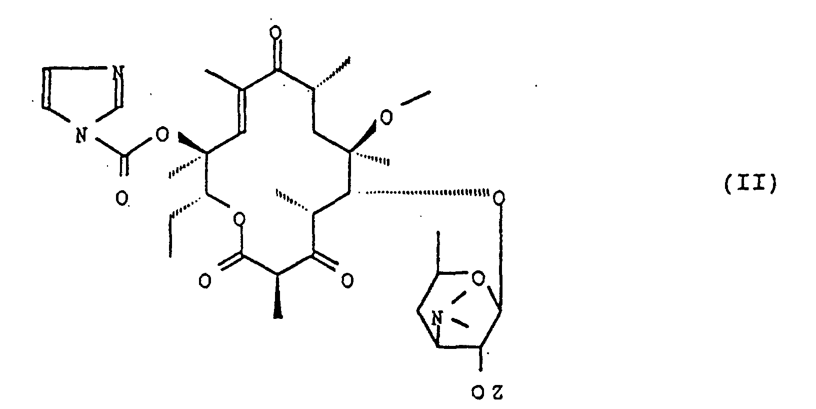 worin Z die in Anspruch 1 angegebene Bedeutung beibehält, der Wirkung einer Verbindung Formel NH₂NHR₂, in der R₂ die in Anspruch 1 gegebene Bedeutung beibehält, unterwirft, um die Verbindung der Formel (I'_{A}) zu erhalten: 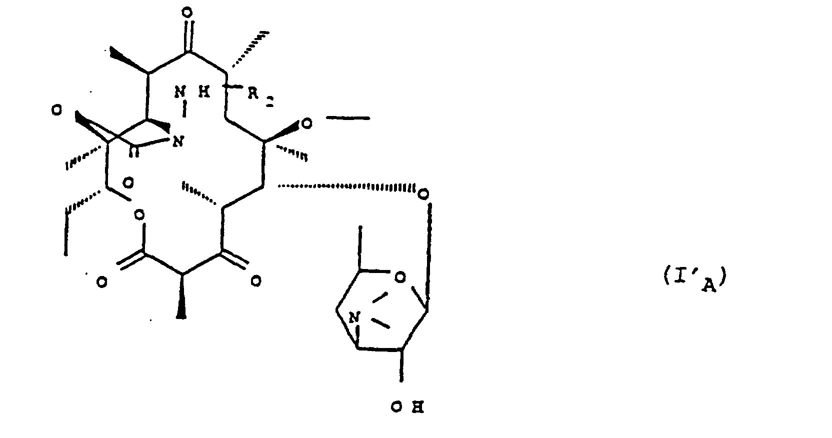 die man, wenn gewünscht, der Wirkung eines Mittels unterwirft, das fähig ist, das Wasserstoffatom der Gruppierung NH durch eine Gruppe R₁, wie sie in Anspruch 1 definiert ist, ausgenommen die Bedeutung Wasserstoff, zu ersetzen, um die entsprechende Verbindung der Formel (I'_{B}) zu erhalten: 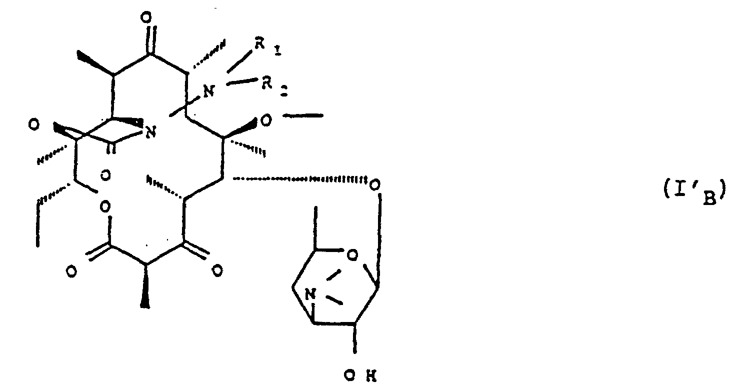 worin R₁ und R₂ die vorstehend angegebene Bedeutung besitzen,
die man, wenn gewünscht, der Wirkung eines Veresterungsmittels für die Gruppierung OH in 2' oder der Wirkung einer Säure, um daraus das Salz zu bilden, unterwirft.

22. Produkte der Formel R'₂CHO oder 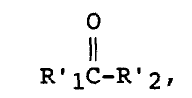 die in Anspruch 20 definiert sind, mit den folgenden Namen:
- 2-Phenyl-5-thiazolpropanal,
- 3-(3-Phenyl-1,2,4-oxadiazol-5-yl)propanal,
- 7-Methoxy-4-chinolinpropanal,
- 2-(3-Pyridinyl)-4-thiazolpropanal
als neue chemische Produkte.

## Claims

1. The compounds of general formula (I) : 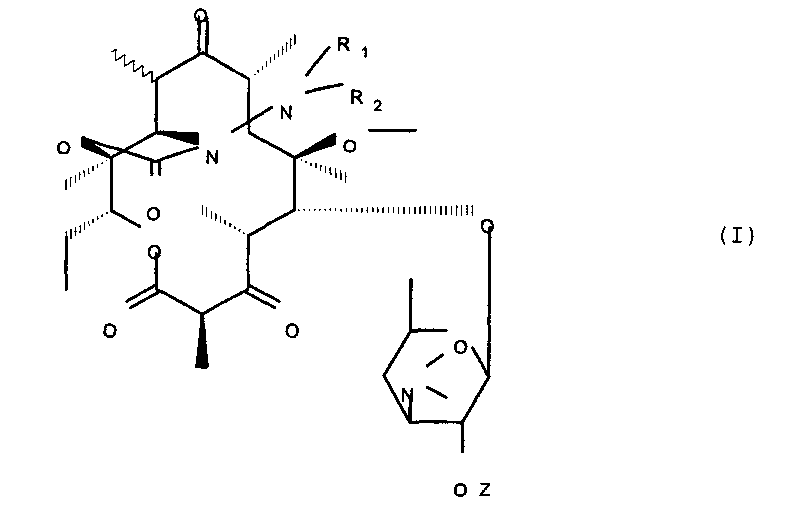 in which:
either R₁ and R₂, identical or different, represent a hydrogen atom, or a hydrocarbon-containing radical containing up to 24 carbon atoms, saturated or unsaturated, optionally interrupted by one or more heteroatoms and optionally carrying one or more functional groups,
or R₁ and R₂ form with the nitrogen atom to which they are linked a heterocycle optionally containing one or more heteroatoms chosen from nitrogen, oxygen and sulphur,
or R₁ and R₂ together form a radical 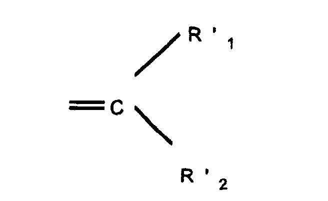 in which R'₁ and R'_{2,} identical or different, represent a hydrogen atom or a hydrocarbon-containing radical containing up to 23 carbon atoms, saturated or unsaturated, optionally interrupted by one or more heteroatoms and optionally carrying one or more functional groups and Z represents a hydrogen atom or the remainder of a carboxylic acid containing up to 18 carbon atoms, the wavy line in position 10 indicating that the methyl can be of R or S configuration, or a mixture of R and S configuration, as well as the addition salts with acids of the compounds of formula (I).

2. The compounds of formula (I) as defined in claim 1 in which Z represents a hydrogen atom.

3. The compounds of formula (I) as defined in claim 1 or 2 in which R₁ represents a hydrogen atom.

4. The compounds of formula (I) as defined in claim 1 or 2 in which R₁ and R₂ each represent a hydrogen atom.

5. The compounds of formula (I) as defined in claim 1 or 2 in which R'₁ represents a hydrogen atom.

6. The compounds of formula (I) as defined in claim 1, 2 or 5 in which R₁ and R₂ together form a:
=CH(CH₂)ₙAr₁
radical in which Ar₁ represents an optionally substituted aryl or heteroaryl radical and n represents an integer which can vary from 0 to 8.

7. The compounds of formula (I) as defined in claim 1, 2 or 5 in which R₁ and R₂ together form a: 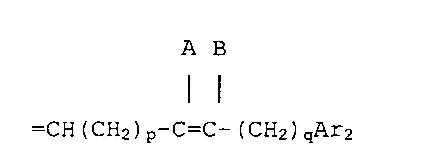 radical in which p and q, identical or different, represent an integer varying from 0 to 6, A and B, identical or different, represent a hydrogen or halogen atom or an alkyl radical containing up to 8 carbon atoms, the geometry of the double bond being E or Z or an E + Z mixture,
or A and B form a third bond with the carbon atoms to which they are linked and Ar₂ represents an optionally substituted mono- or polycyclic, aryl or heteroaryl radical.

8. The compounds of formula (I) as defined in claim 7, in which p and q represent the number 0.

9. The compounds of formula (I) as defined in claim 7 or 8, in which A and B represent a hydrogen atom.

10. The compounds of formula (I), as defined in claim 1, 2 or 3 in which R₂ represents a:
(CH₂)ᵣAr₃
radical in which r represents an integer varying from 0 to 6 and Ar₃ represents an optionally substituted aryl or heteroaryl radical.

11. The compounds of formula (I) as defined in claim 10, in which Ar₃ represents a 4-quinolinyl radical optionally mono or polysubstituted on one and/or the other of the 2 quinoline rings.

12. The compounds of formula (I) as defined in claim 10, in which Ar₃ represents a non substituted 4-quinolinyl radical.

13. The compounds of formula (I) as defined in claim 10, in which Ar₃ represents a 4-quinolinyl radical substituted by a methoxy radical.

14. The compounds of formula(I) as defined in claim 10 in which Ar₃ represents a thiazolyl radical substituted by a pyridyl radical.

15. The compounds of formula (I) as defined in any one of claims 10 to 14, in which r represents an integer varying from 1 to 4.

16. The compounds of formula (I) the names of which follow:
- 11,12-dideoxy 3-de((2,6-dideoxy 3-C-methyl 3-O-methyl alpha L-ribohexopyranosyl) oxy) 6-0-methyl 3-oxo 12,11-(oxycarbonyl (2-(3-(4-quinolinyl) 2-propyl) hydrazono)) erythromycin,
- 11,12-dideoxy 3-de ((2,6-dideoxy 3-C-methyl 3-O-methyl alpha-L-ribohexopyranosyl) oxy) 6-O-methyl 3-oxo 12,11-(oxycarbonyl 2-(3-(7-methoxy-4-quinolinyl) propyl) hydrazono)) erythromycin,
- 11,12-dideoxy 3-de ((2,6-dideoxy 3-C-methyl 3-O-methyl alpha-L-ribohexopyranosyl) oxy) 6-O-methyl 3-oxo 12,11-(oxycarbonyl 2-(3-(2-(3-pyridinyl-4-thiazolyl) propyl) hydrazono)) erythromycin.

17. As medicaments, the compounds of formula (I) as defined in any one of claims 1 to 15 as well as their addition salts with pharmaceutically acceptable acids and bases.

18. As medicaments, the compound defined in claim 16 as well as the addition salts with pharmaceutically acceptable acids.

19. The pharmaceutical compositions containing as active ingredient at least one medicament defined in claim 17 or 18.

20. Preparation process for the compounds of formula (I) as defined in claim 1, **characterized in that** a compound of formula (II) : 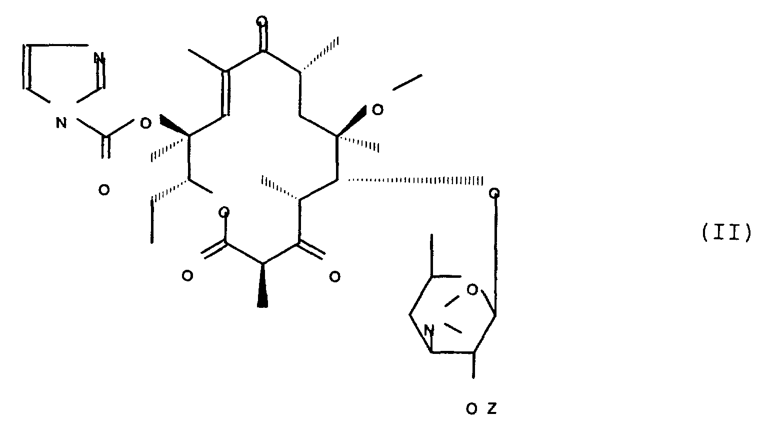 in which Z retains the meaning indicated in claim 1,
is subjected either to the action of hydrazine NH₂NH₂ in order to obtain the compound of formula (I_{A}) : 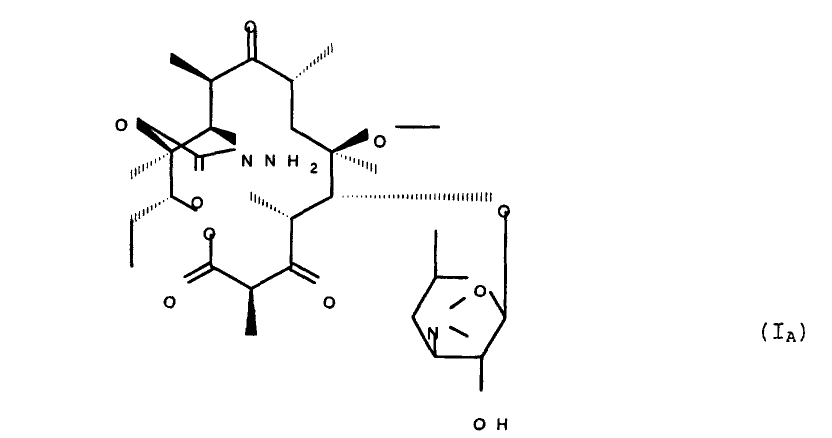 which is subjected, if desired, to the action of an aldehyde R'₂CHO or of a ketone 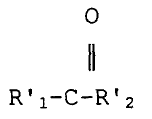 in which R'₁ and R'₂ have the meaning indicated in claim 1, in order to obtain the corresponding compound of formula (I_{B}): 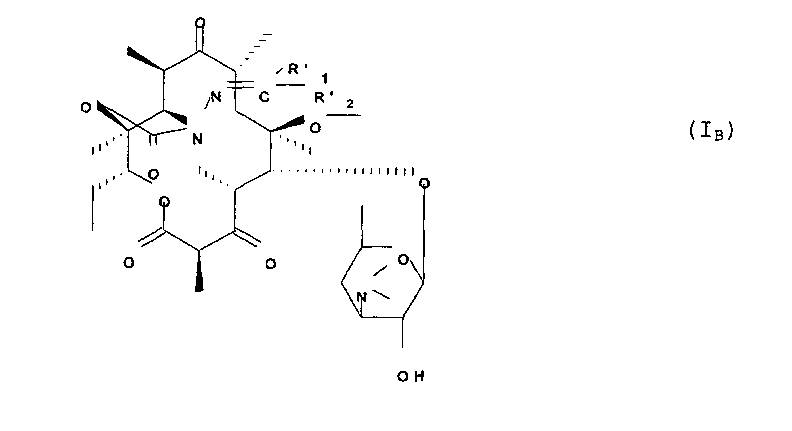 in which R'₁ and R'₂ retain the same meaning as previously, which is subjected if desired to the action of a reducing agent in order to obtain the corresponding compound of formula (I_{C}) : 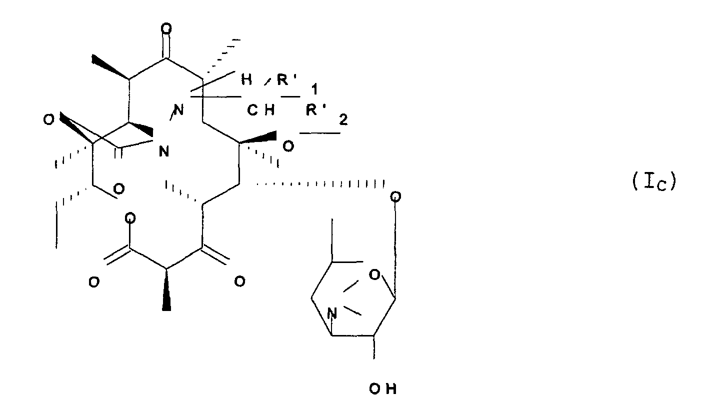 in which R'₁ and R'₂ retain the previous meaning, i.e. a compound of formula (I) in which R₁ represents a hydrogen atom and R₂ represents a CHR'₁-R'₂ radical, then if desired, the compound of formula (I_{C}) is subjected to the action of an agent capable of replacing the hydrogen atom of the NH group by an R₁ group as defined in claim 1, with the exception of the hydrogen value, then if desired, the compounds obtained are subjected to the action of an acid in order to form the salt and/or to the action of an esterification agent of the OH group in position 2'.

21. Process according to claim 20, **characterized in that** the compound of formula (II): 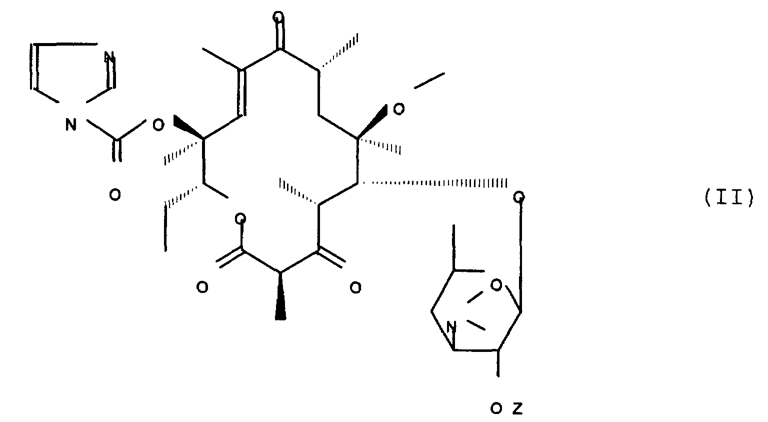 in which Z retains the meaning indicated in claim 1, is subjected to the action of a compound of formula NH₂NHR₂ in which R₂ has the meaning indicated in claim 1 in order to obtain the compound of formula (I'_{A}): 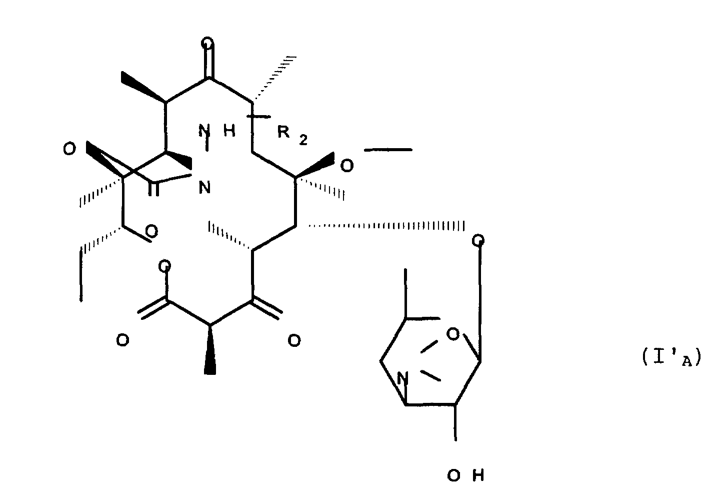 which is subjected, if desired, to the action of an agent capable of replacing the hydrogen atom of the NH group with an R₁ radical as defined in claim 1 with the exception of the hydrogen value in order to obtain the corresponding compound of formula (I'_{B}) : 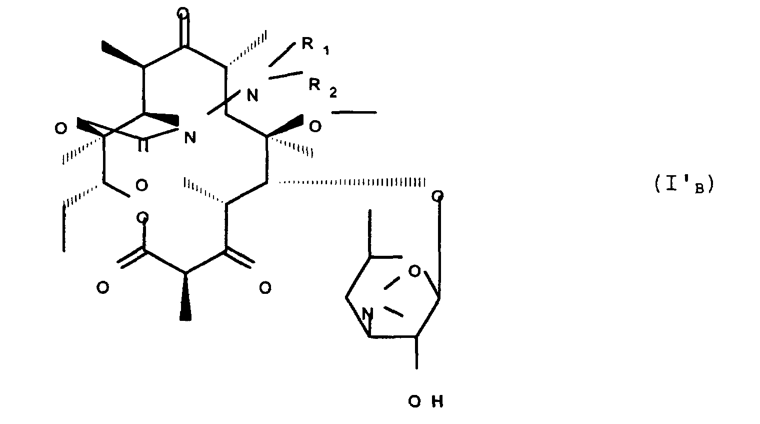 in which R₁ and R₂ have the meaning indicated previously which is subjected, if desired, to the action of an esterification agent of the OH group in position 2' or to the action of an acid in order to form the salt.

22. As new chemical products, the products of formula R'₂CHO or 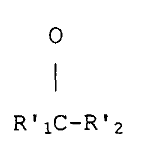 defined in claim 20 the names of which follow:
- 2-phenyl 5-thiazole propanal
- 3-(3-phenyl-1,2,4-oxadiazol 5-yl)propanal
- 7-methoxy 4-quinoline propanal
- 2-(3-pyridinyl) 4-thiazole propanal
